# EUROPEAN PATENT APPLICATION

(11) **EP 4 831 212 A1**
(43) Date of publication of application: **28.10.2026**
(21) Application number: 25744543.7
(22) Date of filing: 20.01.2025
(51) Int. Cl.: C08F 220/60, C08F 8/40, C08F 8/30, A61K 47/59, A61K 45/00, A61P 35/00

(54) **FIBROBLAST ACTIVATION PROTEIN-ALPHA-RESPONSIVE HYDROLYSIS-INDUCED CHARGE-REVERSAL POLYMER-DRUG CONJUGATE, PREPARATION METHOD THEREFOR AND USE THEREOF**

(30) Priority: 26.01.2024 CN 202410111720
(71) Applicant: Revo-Cures (Xiamen) Pharmaceutical Technology Co., Ltd., Xiamen, Fujian 361018 (CN)
(72) Inventor: YU, Qingsong, Xiamen, Fujian 361018 (CN); SUN, Yipei, Xiamen, Fujian 361018 (CN)
(74) Representative: Murgitroyd & Company
(86) International application number: PCT/CN2025/073360
(87) International publication number: WO 2025/157102

(57) **Abstract**

The present disclosure belongs to the technical field of medicines, and particularly relates to a FAP-α-responsive hydrolysis-triggered charge-reversal polymer-drug conjugate as well as a preparation method and use thereof. The FAP-α-responsive hydrolysis-triggered charge-reversal polymer-drug conjugate has the structure represented by Formula (1). The FAP-α-responsive hydrolysis-triggered charge-reversal polymer-drug conjugate provided in the present disclosure exhibits extremely high antitumor efficacy, and can significantly reduce the tumor volume.

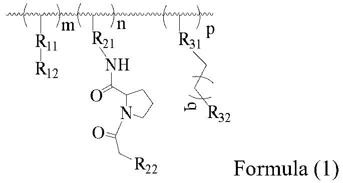

## Description

### TECHNICAL FIELD

The present disclosure belongs to the technical field of medicines, and particularly relates to a fibroblast activation protein-α (FAP-α)-responsive hydrolysis-triggered charge-reversal polymer-drug conjugate as well as a preparation method and use thereof.

### BACKGROUND

According to Cancer Statistics in China, the incidence and mortality of cancer are still gradually increasing year by year with the growth and aging of the population. So far, no highly curative methods have been developed for most cancers. With the advances and development in medicine, more and more treatments are applied clinically, including surgical resection, chemotherapy and radiotherapy. Among them, surgical resection combined with adjuvant chemotherapy is the only method to cure cancers, but not all patients are suitable for surgical resection, as their physical conditions must be taken into account. In addition, surgical resection has the disadvantages of postoperative complications, incomplete surgical resection, and traumatic injuries caused by excessive resection. For patients who cannot undergo surgical resection, only chemotherapy and radiotherapy can be chosen for treatment. Although comprehensive chemotherapy with multiple clinical drugs and continuously developed novel chemotherapeutic drugs have prolonged the survival and improved survival rate of patients, they have the shortcomings such as toxic side effects and unsatisfactory therapeutic efficacy. This not only reduces the quality of life of patients but also limits the therapeutic continuity of clinical drugs, ultimately resulting in some current situations such as limited therapeutic efficacy and excessive side effects.

### SUMMARY

The first objective of the present disclosure is to provide a FAP-α-responsive hydrolysis-triggered charge-reversal polymer-drug conjugate with a favorable antitumor effect.

The second objective of the present disclosure is to provide a preparation method of a FAP-α-responsive hydrolysis-triggered charge-reversal polymer-drug conjugate.

The third objective of the present disclosure is to provide the FAP-α-responsive hydrolysis-triggered charge-reversal polymer-drug conjugate prepared by using the method as described above.

The fourth objective of the present disclosure is to provide use of the FAP-α-responsive hydrolysis-triggered charge-reversal polymer-drug conjugate as described above in the preparation of a drug carrier.

Specifically, the FAP-α-responsive hydrolysis-triggered charge-reversal polymer-drug conjugate provided in the present disclosure has the structure represented by Formula (1):

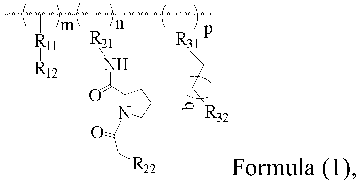

R₁₁ is a linker; R₁₂ is an antitumor drug group; R₂₁ is NH-(CH₂)ₓ-, -O-(CH₂)ₓ-, -CO-(CH₂)ₓ-, -CO-NH-(CH₂)ₓ-, -(CH₂)ₓ-, -(CH₂)ₓ₁O(CH₂)ₓ₂S(CH₂)ₓ₃ or -S-(CH₂)ₓ-, x is 2-6, x1, x2 and x3 are each independently 1-6; R₂₂ is a zwitterionic group; R₃₁ is -NH-, -O-, -OCO-, -NHCO-, -COO-, -CONH-, -S-, -(CH₂)_{z}-O-(CH₂)ₜ-S- or -(CH₂)_{z}-COO-(CH₂)ₜ-S-, q is 1-5, z is 0-5, and t is 1-5; R₃₂ is a zwitterionic group; and m, n and p are molar ratios of three structural units respectively and m:n:p is (5-40%):(10-95%):(0-85%) and m+n+p=100%.

In a preferred embodiment, R₁₁ is selected from at least one of the following structures: 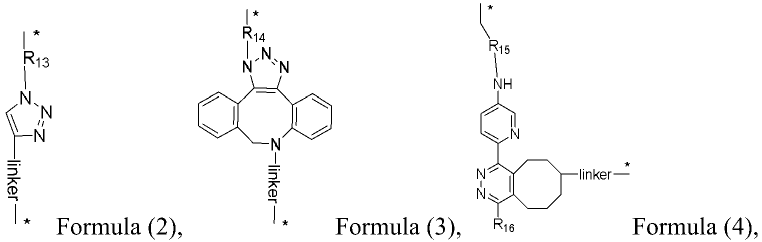 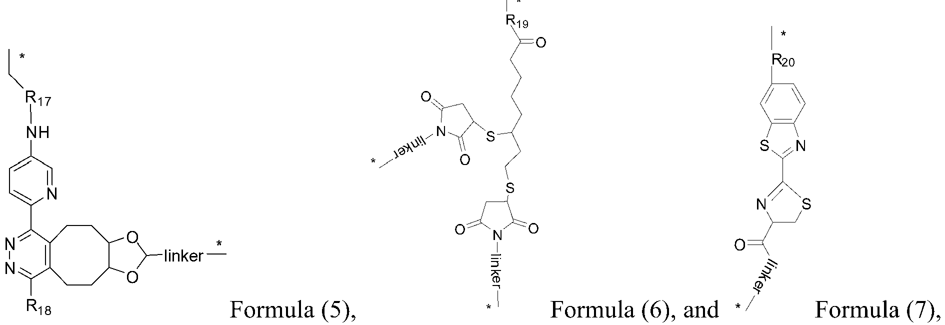
in Formula (2), R₁₃ is an aryl ring, -CO-(CH₂)_{z}-, -O-(CH₂)_{z}-, -NH-(CH₂)_{z}-, -CO-NH-(CH₂)_{z}-, -S-(CH₂)_{z}-, -CO-(CH₂CH₂O)_{z}-, -O-(CH₂CH₂O)_{z}-, -NH-(CH₂CH₂O)_{z}- or -S-(CH₂CH₂O)_{z}-, and z is 1-14;
in Formula (3), R₁₄ is an aryl ring, -CO-(CH₂)_{z}-, -O-(CH₂)_{z}-, -NH-(CH₂)_{z}-, -CO-NH-(CH₂)_{z}-, -S-(CH₂)_{z}-, -CO-(CH₂CH₂O)_{z}-, -O-(CH₂CH₂O)_{z}-, -NH-(CH₂CH₂O)_{z}- or -S-(CH₂CH₂O)_{z}-, and z is 1-14;
in Formula (4), R₁₅ is an aryl ring, -CO-(CH₂)_{z}-CO-, -O-(CH₂)_{z}-CO-, 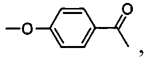 H-(CH₂)_{z}-CO-, -S-(CH₂)_{z}-CO-, -CO-(CH₂CH₂O)_{z}-CO-, -O-(CH₂CH₂O)_{z}-CO-, -NH-(CH₂CH₂O)_{z}-CO- or -S-(CH₂CH₂O)_{z}-CO-, and z is 1-14; and R₁₆ is H or 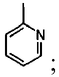
in Formula (5), R₁₇ is an aryl ring, -CO-(CH₂)_{z}-CO-, -O-(CH₂)_{z}-CO-, 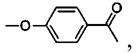 H-(CH₂)_{z}-CO-, -S-(CH₂)_{z}-CO-, -CO-(CH₂CH₂O)_{z}-CO-, -O-(CH₂CH₂O)_{z}-CO-, -NH-(CH₂CH₂O)_{z}-CO- or -S-(CH₂CH₂O)_{z}-CO-, and z is 1-14; and R₁₈ is H or 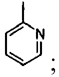
in Formula (6), R₁₉ is an aryl ring, -O-(CH₂)_{z}-O-, -NH-(CH₂)_{z}-NH-, -S-(CH₂)_{z}-NH-, -CO-NH-(CH₂)_{z}- or -S-(CH₂)_{z}-O-, and z is 1-14;
in Formula (7), R₂₀ is an aryl ring, -O-(CH₂)_{z}-O-, -NH-(CH₂)_{z}-O-, -S-(CH₂)_{z}-O-, -CO-NH-(CH₂)_{z}-, -O-(CH₂CH₂O)_{z}-, -NH-(CH₂CH₂O)_{z}- or -S-(CH₂CH₂O)_{z}-, and z is 1-14; and
in the formulas as described above, each linker is independently a group having at least one of reduction, reactive oxygen species, pH and enzyme responsiveness.

In a preferred embodiment, R₂₂ is selected from at least one of the following structures: 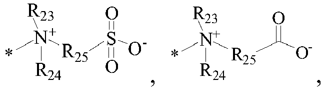 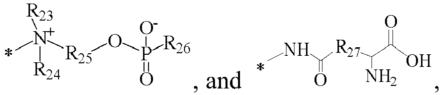 in the formulas described above, R₂₃ and R₂₄ are each independently H or C₁-C₅ alkyl, R₂₅ and R₂₇ are each independently C₁-C₅ alkylene, and R₂₆ is C₁-C₅ alkyl.

In a preferred embodiment, R₃₂ is selected from at least one of the following structures: 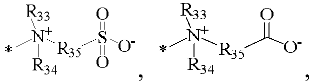 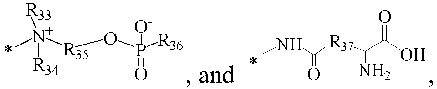 in the formulas described above, R₃₃ and R₃₄ are each independently H or C₁-C₅ alkyl, R₃₅ and R₃₇ are each independently C₁-C₅ alkylene, and R₃₆ is C₁-C₅ alkyl.

In a preferred embodiment, the number-average molecular weight of the polymer-drug conjugate is 10,000-80,000 Da.

In a preferred embodiment, m is 5-40%, n is 10-95%, and p is 0-85%.

In a preferred embodiment, the polymer-drug conjugate is a random copolymer or a block copolymer.

A preparation method of the FAP-α-responsive hydrolysis-triggered charge-reversal polymer-drug conjugate provided in the present disclosure comprises modifying a polymer backbone with a prodrug to obtain the polymer-drug conjugate; and the polymer backbone has the structure represented by Formula (8):

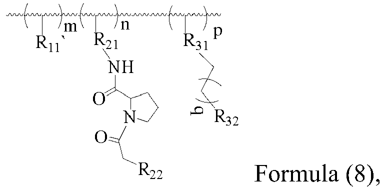

R₁₁' is a linker; R₂₁ is -NH-(CH₂)ₓ-, -O-(CH₂)ₓ-, -CO-(CH₂)ₓ-, -CO-NH-(CH₂)ₓ-, -(CH₂)ₓ-, -(CH₂)ₓ₁O(CH₂)ₓ₂S(CH₂)ₓ₃ or -S-(CH₂)ₓ-, x is 2-6, and x1, x2 and x3 are each independently 1-6; R₂₂ is a zwitterionic group; R₃₁ is -NH-, -O-, -OCO-, -NHCO-, -COO-, -CONH-, -S-, -(CH₂)_{z}-O-(CH₂)ₜ-S- or -(CH₂)_{z}-COO-(CH₂)ₜ-S-, q is 1-5, z is 0-5, and t is 1-5; R₃₂ is a zwitterionic group; and m, n and p are molar ratios of three structural units respectively and m:n:p is (5-40%):(10-95%):(0-85%) and m+n+p=100%.

In a preferred embodiment, in the preparation process of the FAP-α-responsive hydrolysis-triggered charge-reversal polymer-drug conjugate, the modification method is to perform a click chemistry reaction on the R₁₁' in the polymer backbone and the prodrug, and the group for the click chemistry reaction is selected from at least one of azide, trans-cyclooctene, tetrazine, alkyne, dibenzocyclooctyne, bicyclo[6.1.0]nonyne, lipoic acid and maleimide.

In a preferred embodiment, in the preparation process of the FAP-α-responsive hydrolysis-triggered charge-reversal polymer-drug conjugate, R₁₁' is selected from at least one of the following structures: 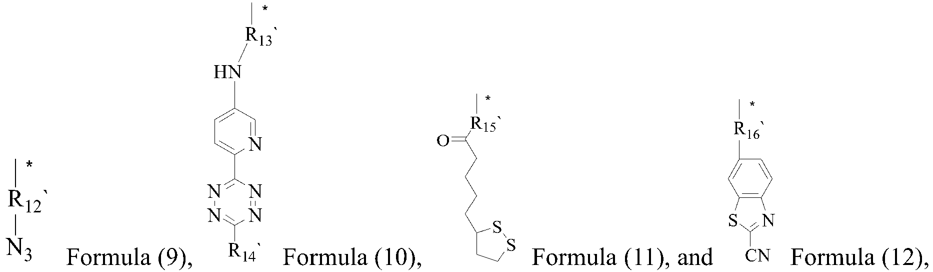
in Formula (9), R₁₂' is an aryl ring, -CO-(CH₂)_{z}-, -O-(CH₂)_{z}-, -NH-(CH₂)_{z}-, -CO-NH-(CH₂)_{z}-, -S-(CH₂)_{z}-, -CO-(CH₂CH₂O)_{z}-, -O-(CH₂CH₂O)_{z}-, -NH-(CH₂CH₂O)_{z}- or -S-(CH₂CH₂O)_{z}-, and z is 1-14;
in Formula (10), R₁₃' is an aryl ring, -CO-(CH₂)_{z}-CO-, -O-(CH₂)_{z}-CO-, 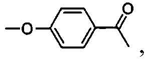 H-(CH₂)_{z}-CO-, -S-(CH₂)_{z}-CO-, -CO-(CH₂CH₂O)_{z}-CO-, -O-(CH₂CH₂O)_{z}-CO-, -NH-(CH₂CH₂O)_{z}-CO- or -S-(CH₂CH₂O)_{z}-CO-, and z is 1-14; and R₁₄' is H or 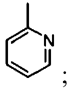
in Formula (11), R₁₅' is an aryl ring, -O-(CH₂)_{z}-O-, -NH-(CH₂)_{z}-NH-, -S-(CH₂)_{z}-NH- or -S-(CH₂)_{z}-O-, and z is 1-14; and
in Formula (12), R₁₆' is an aryl ring, -O-(CH₂)_{z}-O-, -NH-(CH₂)_{z}-O-, -S-(CH₂)_{z}-O-, -CO-NH-(CH₂)_{z}-, -O-(CH₂CH₂O)_{z}-, -NH-(CH₂CH₂O)_{z}- or -S-(CH₂CH₂O)_{z}-, and z is 1-14.

In a preferred embodiment, in the preparation process of the FAP-α-responsive hydrolysis-triggered charge-reversal polymer-drug conjugate, the polymer backbone has at least one of the following structures:

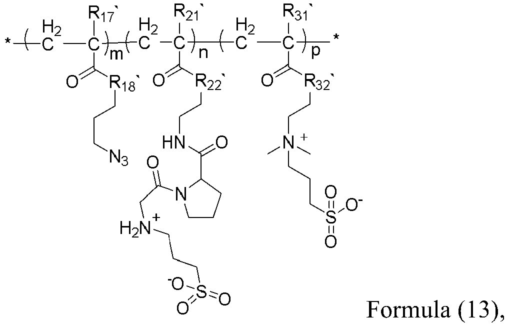

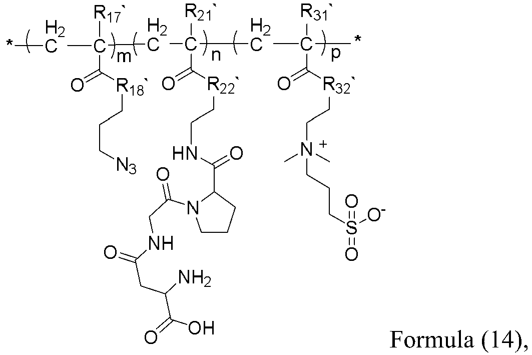

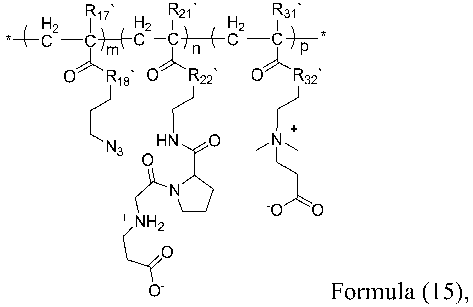

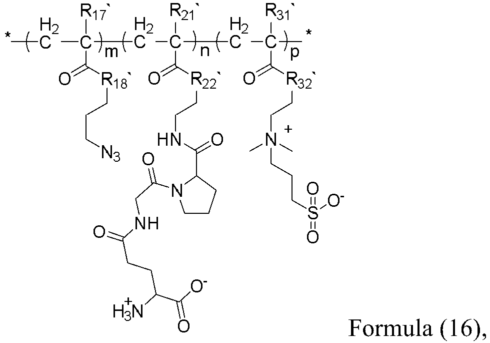

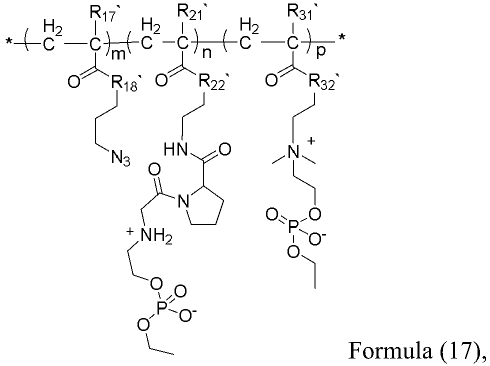

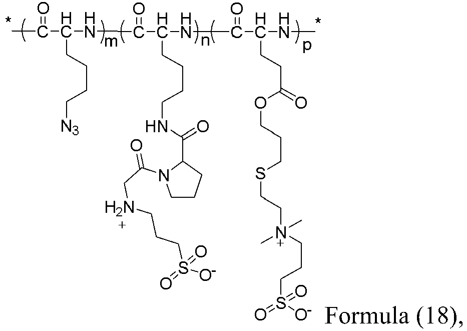

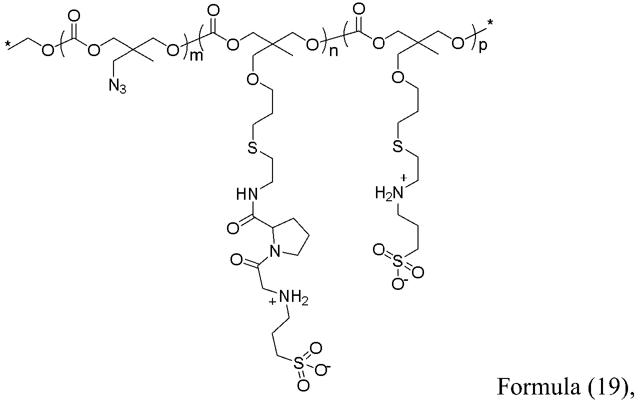

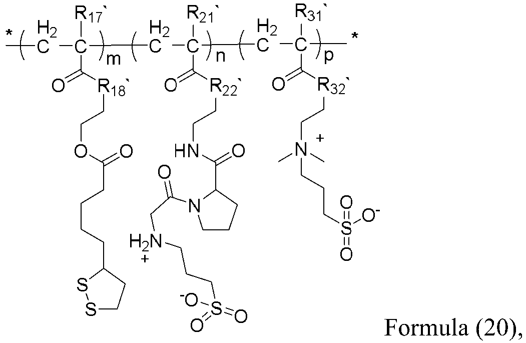

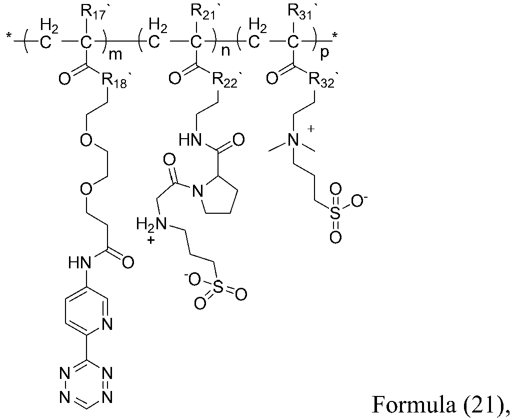

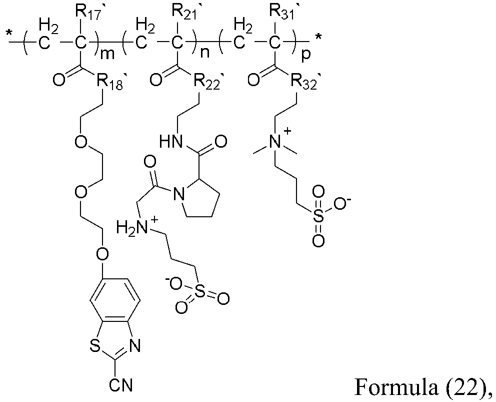

R₁₇', R₂₁' and R₃₁' are each independently H or C₁-C₅ alkyl; and R₁₈', R₂₂' and R₃₂' are each independently O or NH.

In a preferred embodiment, in the preparation process of the FAP-α-responsive hydrolysis-triggered charge-reversal polymer-drug conjugate, the polymer backbone is obtained by random copolymerization or block copolymerization of FAP-α-responsive monomers.

In a preferred embodiment, in the preparation process of the FAP-α-responsive hydrolysis-triggered charge-reversal polymer-drug conjugate, the prodrug has at least one of the following structures: 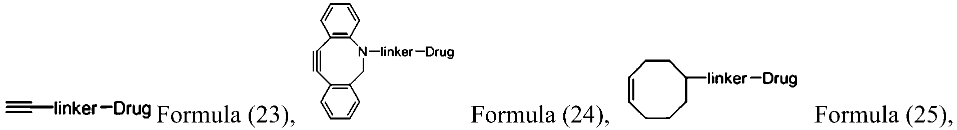 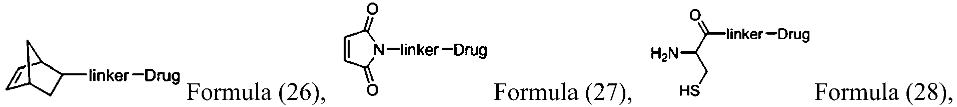 the linker is a group having at least one of reduction, reactive oxygen species, pH and enzyme responsiveness; and Drug is an antitumor drug.

In a preferred embodiment, in the preparation process of the FAP-α-responsive hydrolysis-triggered charge-reversal polymer-drug conjugate, the antitumor drug is selected from at least one of doxorubicin, epirubicin, gemcitabine, cisplatin, carboplatin, paclitaxel, camptothecin, Exatecan, mitomycin C, methotrexate, 7-ethyl-10-hydroxycamptothecin, maytansine, alpha-amanitin, MMAE, MMAF, DM4, calicheamicin, gambogic acid, rhein, vincristine, colchicine, eribulin, Taltobulin, maytansinol, talarostatin A, auristatin E, auristatin F, Piericidin A, ansamitocin P 3, dolastatin 10 and β-Amanitin.

In a preferred embodiment, in the preparation process of the FAP-α-responsive hydrolysis-triggered charge-reversal polymer-drug conjugate, the prodrug has at least one of the following structures: 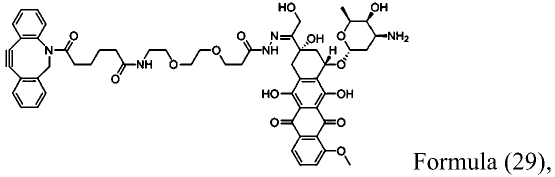 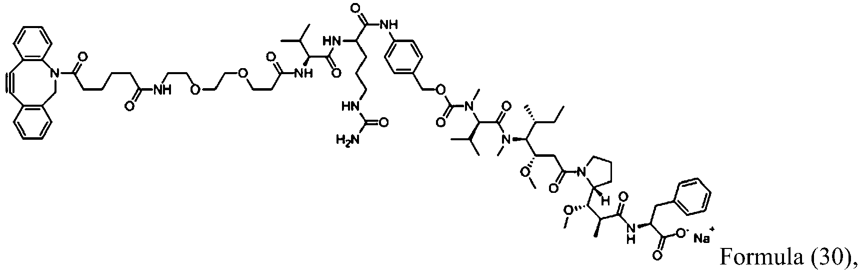 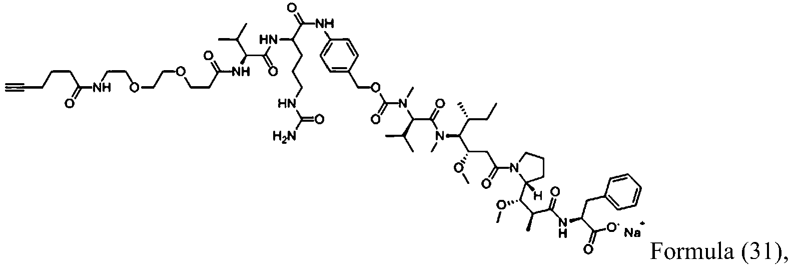 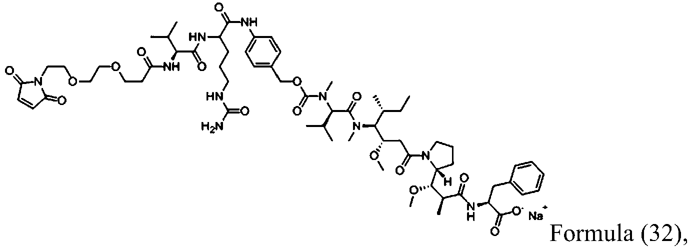 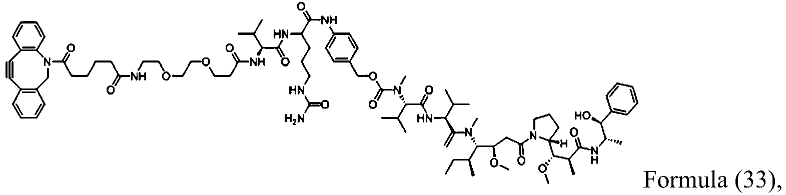 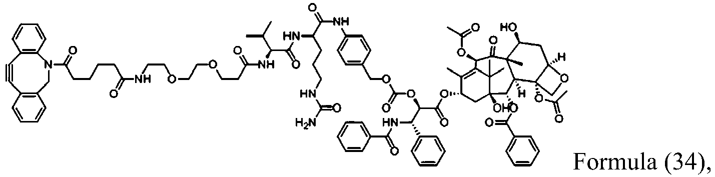 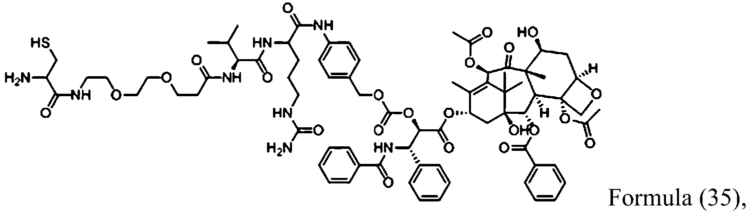 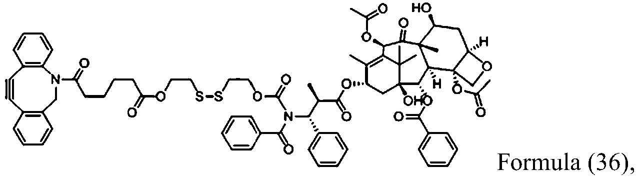 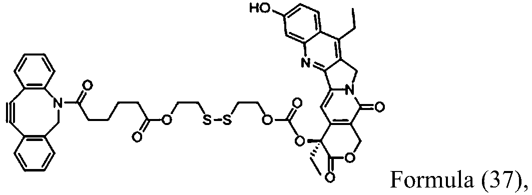 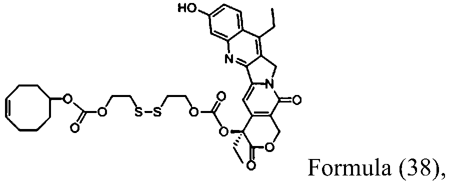 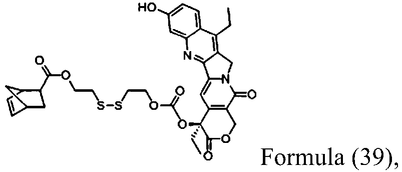 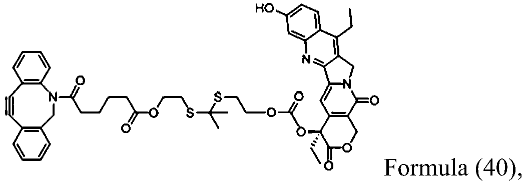 and

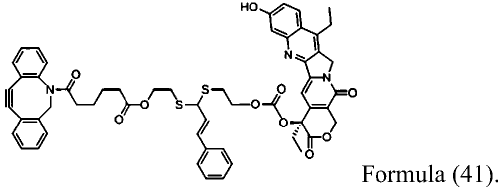

The present disclosure further provides the FAP-α-responsive hydrolysis-triggered charge-reversal polymer-drug conjugate obtained by using the method as described above.

In addition, the present disclosure further provides use of the FAP-α-responsive hydrolysis-triggered charge-reversal polymer-drug conjugate in the preparation of a drug carrier.

One of the theoretical foundations for the development and research of the existing antitumor drug development and research is utilizing the penetrability of the drug in tumor sites. The permeation issue of antitumor drugs in tumor tissues is a key factor of hindering drug efficacy. The tumor tissue has the pathological features of high cell density, dense matrix, high interstitial fluid pressure and the like, and therefore the large size of the antitumor drug makes its diffusion within the tumor extremely difficult. After extravasation from blood vessels, the drug cannot diffuse to cells distant from blood vessels to complete drug delivery, resulting in low therapeutic efficacy.

After extensive and in-depth research, the inventors of the present disclosure have found that cancer-associated fibroblasts (CAFs) in a tumor microenvironment express FAP-α, and some tumors also express FAP-α. Based on this, the inventors of the present disclosure have ingeniously utilized the property of the FAP-α-responsive hydrolysis-triggered charge reversal to synthesize the polymer-drug conjugate having the structure represented by Formula (1) for tumor treatment. The fibroblast activation protein (FAP-α protein) cleaves the polypeptide to induce charge reversal, so as to change the charge from amphoteric to positive, thereby adsorbing and triggering endocytosis, intracellular transport and contralateral exocytosis (transcytosis) in endothelial cells. Since transcytosis is an energy-consuming active transport process in cells, factors such as high osmotic pressure and high cell density that hinder traditional passive diffusion in tumors have no effect on this active transport process. Therefore, the carrier exhibits strong intratumoral penetration ability and can deliver the drug to every cell, thereby achieving high antitumor efficacy and significantly reducing the tumor volume.

### BRIEF DESCRIPTION OF THE DRAWINGS

FIG. 1 is a diagram showing the 4T1 cytotoxicity assay results of monomethyl auristatin E (MMAE) and two drug-conjugated polymers obtained in Example 1-5 and Example 1-7;
FIG. 2 is a diagram showing the pharmacokinetic evaluation results of monomethyl auristatin E (MMAE) and two drug-conjugated polymers obtained in Example 1-5 and Example 1-7;
FIG. 3 is a diagram showing the tumor inhibition assay results of monomethyl auristatin E (MMAE) and two drug-conjugated polymers obtained in Example 1-5 and Example 1-7;
FIG. 4 is a diagram showing the charge reversal results of a (P(DS)-*co*-P(MA-N-GP)-*co*-P(AzMA-CPT)) polymer under the action of FAP-α protein;
FIG. 5 is a diagram showing the cytotoxicity assay results of camptothecin (CPT) and two drug-conjugated polymers obtained in Example 2-5 and Example 2-7;
FIG. 6 is a diagram showing the pharmacokinetic evaluation results of camptothecin (CPT) and two drug-conjugated polymers obtained in Example 2-5 and Example 2-7;
FIG. 7 is a diagram showing the tumor inhibition assay results of camptothecin (CPT) and two drug-conjugated polymers obtained in Example 2-5 and Example 2-7;
FIG. 8 is a diagram showing the cytotoxicity assay results of camptothecin (CPT) and two drug-conjugated polymers obtained in Example 3-7 and Example 3-8;
FIG. 9 is a diagram showing the pharmacokinetic evaluation results of camptothecin (CPT) and two drug-conjugated polymers obtained in Example 3-7 and Example 3-8;
FIG. 10 is a diagram showing the tumor inhibition assay results of camptothecin (CPT) and two drug-conjugated polymers obtained in Example 3-7 and Example 3-8.

### DETAILED DESCRIPTION OF THE EMBODIMENTS

The protein-α (FAP-α)-responsive hydrolysis-triggered charge-reversal polymer-drug conjugate provided in the present disclosure comprises a drug-loading structural unit and a FAP-α-responsive structural unit, and preferably further comprises a hydrophilic structural unit. As long as the polymer-drug conjugate comprises the drug-loading structural unit and the FAP-α-responsive structural unit, it can achieve FAP-α-responsive hydrolysis-triggered charge reversal and exhibit a favorable antitumor effect. When the polymer-drug conjugate further comprises the hydrophilic structural unit, it can effectively prevent the polymer-drug conjugate micelles from being cleared, prolong the action duration of the drug and achieve the purpose of long-term functioning in blood.

In the present disclosure, the FAP-α-responsive hydrolysis-triggered charge-reversal polymer-drug conjugate has the structure represented by Formula (1). Formula (1) is merely used for representing the composition and the content of each structural unit of the FAP-α-responsive hydrolysis-triggered charge-reversal polymer-drug conjugate, rather than a connection relationship between the structural units. The FAP-α-responsive hydrolysis-triggered charge-reversal polymer-drug conjugate can be a random copolymer or a block copolymer, without special limitations. In addition, the molar content of the drug-loading structural unit is represented by m, the molar content of the FAP-α-responsive structural unit is represented by n, and the molar content of the hydrophilic structural unit is represented by p. m, n and p are determined by feed amounts. m:n:p is (5-40%):(10-95%):(0-85%) and m+n+p=100%. Specially, m is 5-40%, such as 5%, 8%, 10%, 12%, 15%, 18%, 20%, 22%, 25%, 28%, 30%, 32%, 35%, 38%, 40%, or any value therebetween. n is 10-95%, such as 10%, 15%, 20%, 25%, 30%, 35%, 40%, 45%, 50%, 55%, 60%, 65%, 70%, 75%, 80%, 85%, 90%, 95%, or any value therebetween. p is 0-85%, such as 0, 0.1%, 0.5%, 1%, 2%, 5%, 10%, 15%, 20%, 25%, 30%, 35%, 40%, 45%, 50%, 55%, 60%, 65%, 70%, 75%, 80%, 85%, or any value therebetween.

In the present disclosure, the drug-loading structural unit has the structure represented by Formula (1-1):

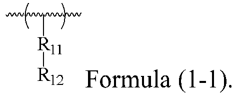

In Formula (1-1), R₁₁ is a linker; and R₁₂ is an antitumor drug group. R₁₁ only needs to bond the antitumor drug to a polymer backbone, and preferably has the structures represented by Formula (2)-Formula (7). In Formula (2)-Formula (7), z is 1-14, such as 1, 2, 3, 4, 5, 6, 8, 10, 12, 14, or any value therebetween; and the linker is a group having at least one of reduction, reactive oxygen species, pH and enzyme responsiveness. When the linker is the group having at least one of reduction, reactive oxygen species, pH and enzyme responsiveness, the corresponding polymer-drug conjugate also has at least one property of reduction, reactive oxygen species, pH and enzyme responsiveness, with stronger universality. R₁₂ is an antitumor drug group. Specific examples of the antitumor drug include, but are not limited to, at least one of doxorubicin, epirubicin, gemcitabine, cisplatin, carboplatin, paclitaxel, camptothecin, Exatecan, mitomycin C, methotrexate, 7-ethyl-10-hydroxycamptothecin, maytansine, alpha-amanitin, MMAE, MMAF, DM4, calicheamicin, gambogic acid, rhein, vincristine, colchicine, eribulin, Taltobulin, maytansinol, talarostatin A, auristatin E, auristatin F, Piericidin A, ansamitocin P 3, dolastatin 10 and β-Amanitin.

In the present disclosure, the FAP-α-responsive structural unit has the structure represented by Formula (1-2):

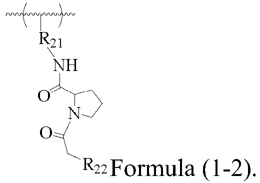

In Formula (1-2), R₂₁ is -NH-(CH₂)ₓ-, -O-(CH₂)ₓ-, -CO-(CH₂)ₓ-, -CO-NH-(CH₂)ₓ-, -(CH₂)ₓ-, -(CH₂)ₓ₁O(CH₂)ₓ₂S(CH₂)ₓ₃ or -S-(CH₂)ₓ-, x is 2-6 (such as 2, 3, 4, 5, 6, or any value therebetween), and x1, x2 and x3 are each independently 1-6 (such as 1, 2, 3, 4, 5, 6, or any value therebetween). R₂₂ is a zwitterionic group, i.e., a group with positive and negative charges. Specifically, R₂₂ is preferably selected from at least one of the following structures:

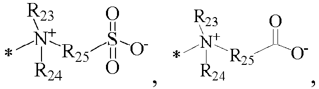

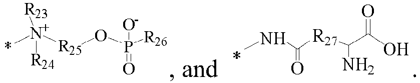

In the formula described above, R₂₃ and R₂₄ are each independently H or C₁-C₅ alkyl, R₂₅ and R₂₇ are each independently C₁-C₅ alkylene, and R₂₆ is C₁-C₅ alkyl. Specific examples of the C₁-C₅ alkyl include, but are not limited to, methyl, ethyl, n-propyl, isopropyl, n-butyl, sec-butyl, isobutyl, tert-butyl, n-pentyl, isopentyl, tert-pentyl or neopentyl. Specific examples of the C₁-C₅ alkylene include, but are not limited to, methylene, ethylene, propylene/n-propylene, isopropylene, n-butylene, sec-butylene, isobutylene, tert-butylene, n-pentylene, isopentylene, tert-pentylene or neopentylene.

In the present disclosure, the hydrophilic structural unit has the structure represented by Formula (1-3):

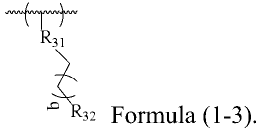

In Formula (1-3), R₃₁ is -NH-, -O-, -OCO-, -NHCO-, -COO-, -CONH-, -S-, -(CH₂)_{z}-O-(CH₂)ₜ-S- or -(CH₂)_{z}-COO-(CH₂)ₜ-S-; q is 1-5, such as 1, 2, 3, 4, 5, or any value therebetween; z is 0-5, such as 0, 1, 2, 3, 4, 5, or any value therebetween; and t is 1-5, such as 1, 2, 3, 4, 5, or any value therebetween. R₃₂ is a zwitterionic group, i.e., a group with positive and negative charges. Specifically, R₃₂ is preferably selected from at least one of the following structures: 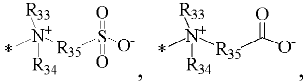 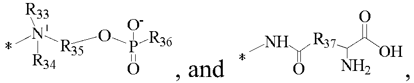 in the formulas described above, R₃₃ and R₃₄ are each independently H or C₁-C₅ alkyl, R₃₅ and R₃₇ are each independently C₁-C₅ alkylene, and R₃₆ is C₁-C₅ alkyl. Specific examples of the C₁-C₅ alkyl include, but are not limited to, methyl, ethyl, n-propyl, isopropyl, n-butyl, sec-butyl, isobutyl, tert-butyl, n-pentyl, isopentyl, tert-pentyl or neopentyl. Specific examples of the C₁-C₅ alkylene include, but are not limited to, methylene, ethylene, propylene/n-propylene, isopropylene, n-butylene, sec-butylene, isobutylene, tert-butylene, n-pentylene, isopentylene, tert-pentylene or neopentylene.

In the present disclosure, the main chain of the FAP-α-responsive hydrolysis-triggered charge-reversal polymer-drug conjugate can be selected from at least one of poly(meth)acrylate, poly(meth)acrylamide, poly(amino acid), polycarbonate and the like, without special limitations.

The preparation method of the FAP-α-responsive hydrolysis-triggered charge-reversal polymer-drug conjugate provided in the present disclosure comprises modifying the prodrug onto the polymer backbone. The polymer backbone is obtained by copolymerization of monomers. The method of introducing the drug via post-polymerization modification can avoid the issue that many drug monomers cannot be directly polymerized, thereby preventing their unsuccessful incorporation into polymers. The modification method is to perform a click chemistry reaction on the R₁₁' in the polymer backbone and the prodrug, and the group for the click chemistry reaction is selected from at least one of azide, trans-cyclooctene, tetrazine, alkyne, dibenzocyclooctyne, bicyclo[6.1.0]nonyne, lipoic acid and maleimide.

To further understand the present disclosure, the present disclosure will be described in detail with reference to the embodiments, but the present disclosure is not limited to these embodiments or application examples. Changes, substitutions, combinations, simplifications, etc., made by those skilled in the art under the core guiding concept of the present disclosure shall all fall within the scope of protection of the present disclosure.

### Example 1-1: Synthesis of monomer AzMA

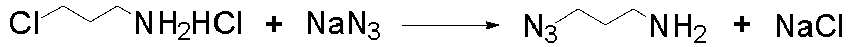

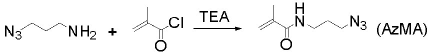

3-chloropropylamine hydrochloride (1.2 g, 9.2 mmol) was dissolved into 30 mL of deionized water. After complete dissolution, sodium azide (2.25 g, 34.6 mmol) was added to the above solution, and the mixture reacted for 24 h at 100°C in the dark. After completion of the reaction, the reaction product was slowly cooled to room temperature, potassium hydroxide was added until a potassium hydroxide solid was precipitated out, and meanwhile oil droplets were seen on the liquid surface. Subsequently, the mixture was extracted with diethyl ether three times, dried over anhydrous MgSO₄, and subjected to rotary evaporation to obtain an intermediate product.

The intermediate product (0.94 g, 9.1 mmol), 1.89 mL of triethylamine (1.38 g, 13.7 mmol) and 4-tert-butylcatechol (3.32 mg, 0.002 mmol) were dissolved into 20 mL of dichloromethane (DCM), and then 1.94 mL of methacryloyl chloride (1.18 g, 10 mmol) was added at 0°C. The mixture reacted at room temperature overnight, concentrated under reduced pressure at low temperature, washed with pure water three times, dried over anhydrous MgSO₄, and purified by silica gel column chromatography (a mobile phase: dichloromethane/methanol = 100:1 (V/V)) to obtain a product, i.e., AzMA.

### Example 1-2: Synthesis of prodrug DBCO-Val-Cit-PABC-MMAE

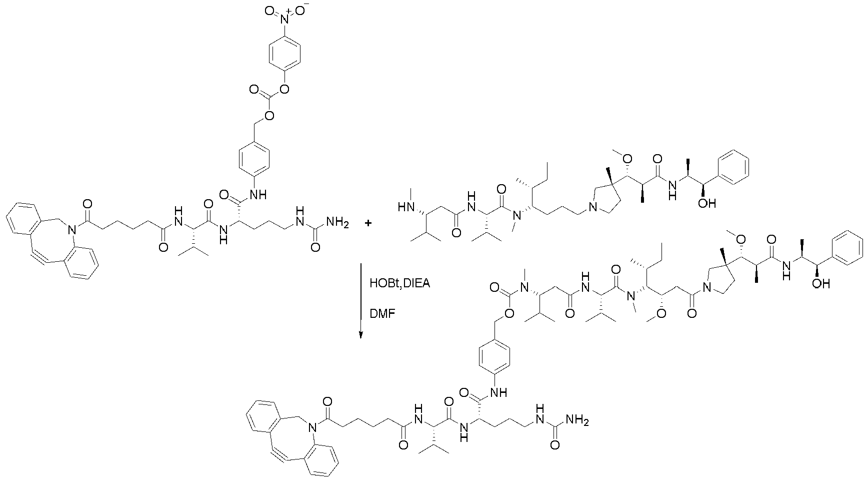

MMAE (0.011g, 0.014mmol), DBCO-Val-Cit-PABC-PNP (0.023 g, 0.027 mmol) and N-hydroxybenzotriazole (HOBt) (0.004 g, 0.030 mmol) were dissolved into N,N-dimethylformamide (DMF) (0.3 mL), and then N,N-diisopropylethylamine (DIEA) (0.011 mL) was added. The mixture was stirred for 16 h at room temperature, followed by removal of DMF under reduced pressure. The crude reaction product was purified by silica gel column chromatography (a mobile phase: dichloromethane/methanol = 13:1 (V/V)) to obtain a white solid (DBCO-Val-Cit-PABC-MMAE, 9.2 mg, yield 49%).

The chemical shift of DBCO-Val-Cit-PABC-MMAE: ¹H-NMR(CDCl₃), δ(ppm): 0.9-1.1(CH₂CH₃, CCH₃,CCH₃); 1.3-1.5(C(C*H*₃)₃); 1.7-2.4(CCH₂CH₂C, COCCH₂CH₃, CHCH₂, CHCH₂CH₂); 2.8-3.0(NCH₃); 3.2-3.6(NCH₂CH₃,OCH₃); 3.7-4.2(COCH₂NH, COOCH₂, COOCH₂); 4.2-4.6(OCOOCH₂, NHCOCH); 5.2-5.9(NCH₂C, COOCH₂C); 6.0-7.0,7.5-8.0(NHCO); 7.0-7.7(ph).

### Example 1-3: Synthesis of FAP-α-responsive monomer MA-N-GP

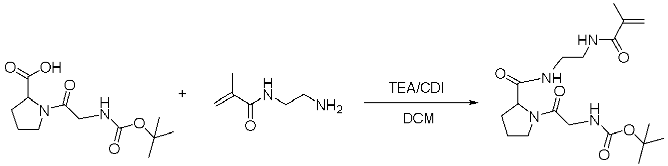

Under argon, Boc-glycine-L-proline (Boc-GP, 2.50 g, 4.6 mmol) and carbonyldiimidazole (CDI, 1.50 g, 4.6 mmol) were added to a flask, followed by addition of 25 mL of anhydrous dichloromethane, and then the above mixture was sufficiently stirred until it was completely dissolved, and then activated under stirring at room temperature for 2 h. 2-aminoethyl methacrylamide hydrochloride (MA-N-NH₂·HCl, 0.92 g, 5.5 mmol) and triethylamine (TEA, 4.7 mL, 16.6 mmol) were added to another flask, followed by addition of 30 mL of anhydrous dichloromethane, and then the above mixture sufficiently stirred at room temperature for 2 h to obtain a MA-N-NH₂ reaction solution. The reaction solution activated in the first flask was dropwise added to the MA-N-NH₂ reaction solution by using a constant-pressure funnel, and then the above mixture reacted under stirring at room temperature for 24 h. After completion of the reaction, the resulting product solution was washed with 0.5 M HCL three times. The washed product solution was dried over anhydrous magnesium sulfate, subjected to suction filtration, concentrated via rotary evaporation and purified by silica gel column chromatography using ethyl acetate as a mobile phase. The second fraction was collected, and underwent rotary evaporation to remove the mobile phase to obtain a white solid powder (monomer MA-N-GP, 1.6 g, yield 47%).

### Example 1-4: Synthesis of FAP-α-responsive polymer

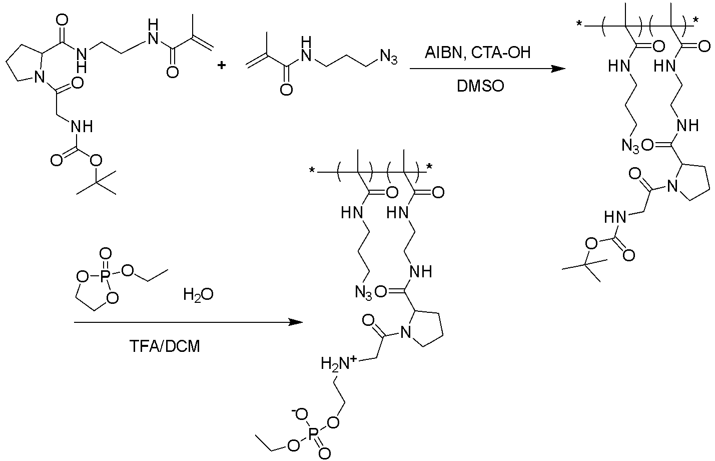

(1) An RAFT polymerization method was adopted, and a clean Schlenk tube was dried in an oven at 120°C. After completion of the drying, the reaction tube was taken out and cooled to room temperature. A paper funnel was made of filter paper and placed at the mouth of the tube. The monomers MA-N-GP (500 mg, 1.28 mmol) and AzMA (70 mg, 0.42 mmol) as well as an initiator azobisisobutyronitrile (AIBN, 1.6 mg, 0.01 mmol) were added to the bottom of the reaction tube through the paper funnel. A dithioester chain transfer agent (CTA-OH, 6.2 mg, 0.02 mmol) was weighed and dissolved in 800 µL of ultra-dry dimethyl sulfoxide (DMSO), the solution obtained after dissolution was added to the bottom of the Schlenk tube, and then the mixture was dissolved under stirring. Subsequently, the mixture was subjected to three cycles of liquid nitrogen freezing - evacuation for 10 min - argon filling - thawing utilizing a vacuum evacuation device. The reaction tube filled with argon was then placed in an oil bath at 70°C for 48 h of reaction. After completion of the reaction, the reaction solution was dropwise added to 15 mL of ethyl acetate to precipitate out an orange-yellow solid product. The resulting suspension was centrifuged and then the supernatant was discarded. The precipitation procedure was repeated twice to remove unreacted monomers and the DMSO solvent. The obtained solid was dried for 24 h to obtain a prepolymer.
(2) 80 mg of prepolymer was dissolved in 2 mL of dichloromethane. After complete dissolution, 400 µL of trifluoroacetic acid was added in an ice bath, and then the mixture reacted under stirring in the ice bath for 1 h to remove a Boc protecting group. After completion of the reaction, the reaction product was subjected to rotary evaporation to remove dichloromethane and trifluoroacetic acid. Then, the residue was dried in a vacuum oven at room temperature for 4 h. 4 mL of ultrapure water was added to the dried flask, and then the mixture was stirred to be dissolved. The pH of the above solution was adjusted to 8.0 using a saturated sodium bicarbonate solution. 50 mg of 2-ethoxy-2-oxide-1,3,2-dioxaphospholane was weighed and dissolved in 1 mL of ultrapure water, and then the above solution was added to the reaction solution. The above mixture reacted under stirring in a water bath at 26°C for 36 h. After completion of the reaction, the reaction solution was transferred into a dialysis bag (MWCO=1000 Da) and dialyzed against ultrapure water for 24 h. Water was changed every 6 h. The whole procedure was carried out in the dark. The polymer solution obtained by dialysis was the FAP-α-responsive polymer which was directly stored in a refrigerator at 4°C.

The chemical shift of FAP-α-responsive polymer: ¹H-NMR(MeOD), δ(ppm): 0.7-1.0(CH₂CH₃); 1.6-2.1(CCH₂CH₂C, CH₂CH₃); 2.8-3.2(N(CH₂)₂, N₃CH₂); 3.2-3.4(N(CH₃)₃); 3.6-3.8(CH₂N(CH₃)₃); 4.0-4.4((NCH₂CH₂)₂, COOCH₂CH₂O, POCH₂); 6.0-7.0(NHCO).

### Example 1-5: Synthesis of FAP-α-responsive polymer P(MA-N-GPP)-co-P(AzMA-MMAE) containing MMAE drug linker

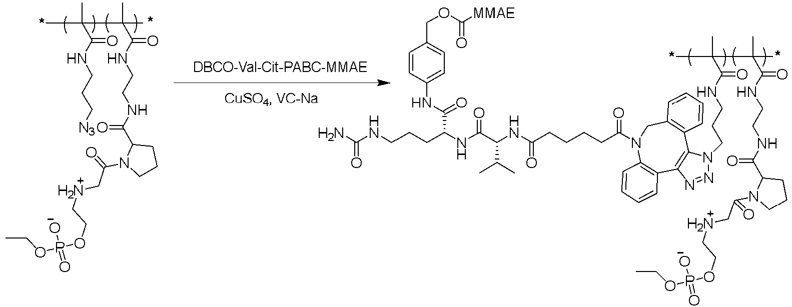

The FAP-α-responsive polymer obtained in Example 1-4 (67 mg, 6.67 mmol), DBCO-Val-Cit-PABC-MMAE (110 mg, 66.7 mmol) and PMDETA (1.772 µg, 20 µL, 0.001 mmol) were weighed and dissolved in 0.3 mL of DMF/water mixed solvent (DMF: water = 7:3, v/v). After evacuation twice under argon, copper sulfate pentahydrate and vitamin C (copper sulfate pentahydrate: vitamin C = 1:5, w/w) were added, followed by evacuation three more times. The mixture reacted at 45°C for 48 h. After completion of the reaction, the reaction product was precipitated three times with ice-cold diethyl ether, dialyzed against DMSO and deionized water in sequence, and finally lyophilized to obtain a product, i.e., the FAP-α-responsive polymer P(MA-N-GPP)-co-P(AzMA-MMAE) containing the MMAE drug linker.

The chemical shift of the FAP-α-responsive polymer P(MA-N-GPP)-co-P(AzMA-MMAE) containing the MMAE drug linker: ¹H-NMR(CDCl₃), δ(ppm): 0.9-1.1(CH₂CH₃, CCH₃, CCH₃); 1.3-1.5(C(C*H*₃)₃); 1.7-2.4(CCH₂CH₂C, COCCH₂CH₃, CHCH₂, CHCH₂CH₂); 2.8-3.0(NCH₃); 3.2-3.6(NCH₂CH₃, OCH₃); 3.7-4.2(COCH₂NH, COOCH₂); 4.2-4.6(OCOOCH₂, NHCOCH, POCH₂); 5.2-5.9(NCH₂C); 6.0-7.0,7.5-8.0(NHCO); 7.0-7.7(ph).

### Example 1-6: Synthesis of FAP-α-responsive polymer containing hydrophilic monomers

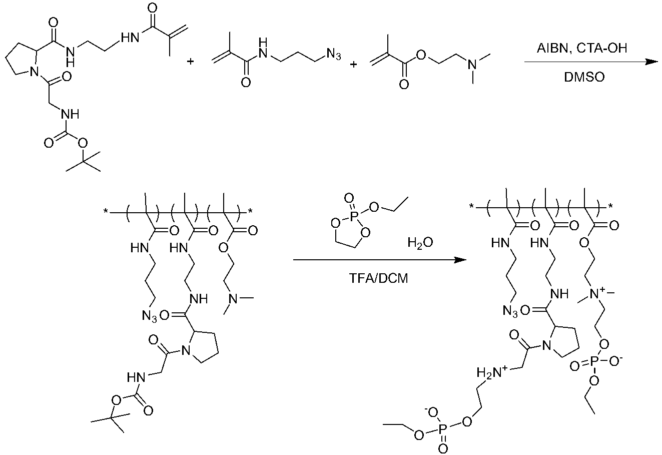

(1) An RAFT polymerization method was adopted, and a clean Schlenk tube was dried in an oven at 120°C. After completion of the drying, the reaction tube was taken out and cooled to room temperature. A paper funnel was rolled from filter paper and placed on the mouth of the tube. Monomers MA-N-GP (500 mg, 1.28 mmol), AzMA (70 mg, 0.42 mmol) and dimethylaminoethyl methacrylate (DMAEMA, 294 mg, 1.28 mmol) as well as an initiator 2,2'-azobisisobutyronitrile (AIBN, 1.6 mg, 0.01 mmol) were weighed and added to the bottom of the reaction tube through a paper funnel. The chain transfer agent CTA-OH (6.2 mg, 0.02 mmol) was weighed and dissolved in 800 µL of ultra-dry dimethyl sulfoxide (DMSO), and the resulting solution was added to the bottom of the Schlenk tube under stirring to achieve complete dissolution. Subsequently, the mixture was subjected to three cycles of liquid nitrogen freezing - evacuation for 10 min - argon filling - thawing utilizing a vacuum evacuation device. After that, the reaction tube filled with argon was placed in an oil bath at 70°C for 48 h of reaction. After completion of the reaction, the reaction solution was added dropwise into 15 mL of ethyl acetate to precipitate an orange-yellow solid product. The resulting suspension was concentrated and the supernatant was discarded. The precipitation procedure was repeated twice to remove unreacted monomers and the DMSO solvent. The obtained solid was dried for 24 h to obtain a prepolymer.
(2) 80 mg of prepolymer was dissolved in 2 mL of dichloromethane. After complete dissolution, 400 µL of trifluoroacetic acid was added in an ice bath, and the mixture reacted under stirring in the ice bath for 1 h to remove a Boc protecting group. After completion of the reaction, the reaction product was subjected to rotary evaporation to remove dichloromethane and trifluoroacetic acid. Then, the residue was dried in a vacuum oven at room temperature for 4 h. 4 mL of ultrapure water was added to the dried flask, and the mixture was dissolved under stirring. The pH of the solution was adjusted to 8.0 using a saturated sodium bicarbonate solution. 50 mg of 2-ethoxy-2-oxide-1,3,2-dioxaphospholane was weighed and dissolved in 1 mL of ultrapure water, and the resulting solution was added to the reaction solution, followed reaction under stirring in a water bath at 26°C. After completion of the reaction, the reaction solution was transferred into a dialysis bag (MWCO=1000 Da) and dialyzed against ultrapure water for 24 h. Water was changed every 6 h. The whole process was carried out in the dark. The polymer solution obtained by dialysis was an amphiphilic FAP-α-responsive polymer which was directly stored in a refrigerator at 4°C.

### Example 1-7: Synthesis of FAP-α-responsive polymer P(DP)-co-P(MA-N-GPP)-co-P(AzMA-MMAE) containing MMAE drug linkers and hydrophilic monomers

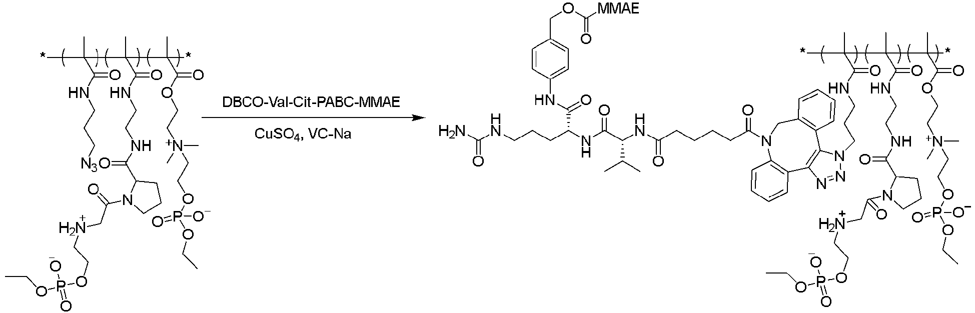

The FAP-α-responsive polymer containing hydrophilic monomers obtained in Example 1-6 (100 mg, 6.67 mmol), DBCO-Val-Cit-PABC-MMAE (110 mg, 66.7 mmol) and PMDETA (1.772 µg, 20 µL, 0.001 mmol) were weighed and dissolved in 0.3 mL of DMF/water mixed solvent (DMF: water = 7:3, v/v). After evacuation twice under argon, copper sulfate pentahydrate and vitamin C (copper sulfate pentahydrate: vitamin C = 1:5, w/w) were added, followed by evacuation three more times. The mixture reacted at 45°C for 48 h. After completion of the reaction, the reaction product was precipitated three times with ice-cold diethyl ether, dialyzed against DMSO and deionized water in sequence, and lyophilized to obtain a product, i.e., the FAP-α-responsive polymer P(DP)-*co*-P(MA-N-GPP)-*co*-P(AzMA-MMAE) containing MMAE drug linkers and hydrophilic monomers.

### Application example 1-1

### (1) Preparation of polymer injection

P(MA-N-GPP)-*co*-P(AzMA-MMAE) and P(DP)-*co*-P(MA-N-GPP)-*co*-P(AzMA-MMAE) were respectively dissolved in normal saline and polymer injections were prepared at an MMAE-equivalent concentration of 0.2 mg/kg body weight to obtain homogeneous, clear and transparent polymer injections.

### (2) Cytotoxicity assay

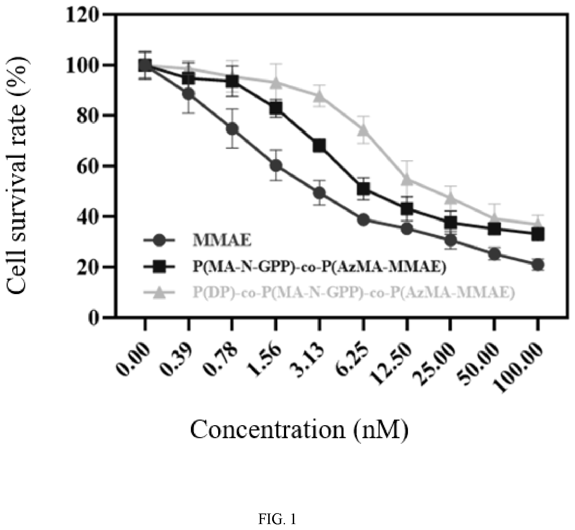

The 4T1 cytotoxicity assay was performed on monomethyl auristatin E (MMAE) and the two polymer injections. The results are shown in FIG. 1. As can be seen from FIG. 1, the drug-conjugated polymer (P(DP)-*co*-P(MA-N-GPP)-*co*-P(AzMA-MMAE)) containing hydrophilic monomers has relatively strong cytotoxicity against 4T1 cells, but it is difficult to enter the cell membrane due to its resistance to protein adsorption. Therefore, compared with the polymer P(MA-N-GPP)-*co*-P(AzMA-MMAE)) containing no hydrophilic monomers and a free drug (monomethyl auristatin E), the drug-conjugated polymer (P(DP)-*co*-P(MA-N-GPP)-*co*-P(AzMA-MMAE)) containing hydrophilic monomers has lower cytotoxicity. The cytotoxicity assay results show that both the synthesized two polymer-drug conjugates have a 4T1 killing effect, and possess remarkable cytotoxicity.

### (3) Pharmacokinetic evaluation

2 mL of two drug-conjugated polymer solutions obtained in Example 1-5 and Example 1-7 were separately taken, and then 200 µL of 1 mg/mL Cy5.5-alkyne solution was added. After evacuation twice under argon, copper sulfate pentahydrate and vitamin C (copper sulfate pentahydrate: vitamin C = 1:5, w/w) were added, followed by reaction under stirring at room temperature for 24 h. After completion of the reaction, the mixture was first dialyzed against methanol for 3 h, followed by dialysis against ultrapure water for 24 h to obtain Cy5.5-labeled polymer solutions.

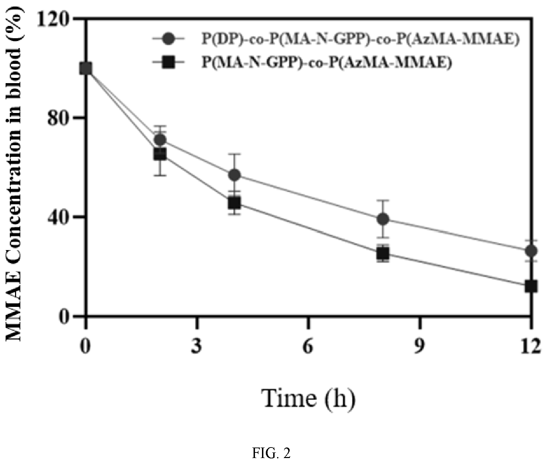

After the mice were acclimated to the environment, 6 mice were divided into two groups with 3 mice per group. The Cy5.5-labeled polymer solutions were intravenously injected into the tail veins of the mice at a dose of 5 mg/kg. After the serum clearance half-life t₁/₂, blood samples (approximately 20 µL) were collected from the capillaries around the eyes of the mice, and the fluorescence intensity was measured. The results are shown in FIG. 2. As can be seen from FIG. 2, the t_{1/2} of P(MA-N-GP)-*co*-P(AzMA-MMAE) is 3.6 h, and the t_{1/2} of P(DP)-*co*-P(MA-N-GPP)-*co*-P(AzMA-CPT) is 5.6 h, indicating that the polymerization with the hydrophilic monomers can effectively prevent the micelles from being cleared, thereby achieving the purpose of increasing the blood circulation time and ensuring sufficient long blood circulation.

### (4) Tumor inhibition assay

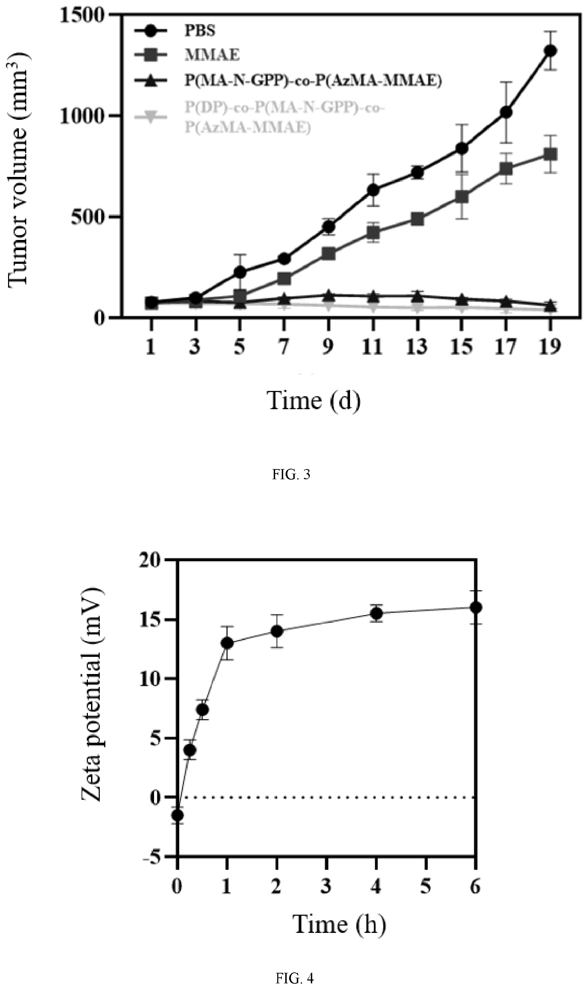

The tumor inhibitory effect of MMAE-conjugated polymers in 4T1 breast cancer tumor-bearing mice was investigated. The assay was initiated when the tumor volume reached approximately 100 mm³. MMAE (free drug) and two drug-conjugated polymer injections were prepared. A PBS buffer was used as a blank group. The drugs were administered via tail vein injection at an MMAE-equivalent dose of 0.2 mg/kg, with an injection volume of 100 µL per mouse each time. The mice were administered on days 1, 4, 7, 10 and 13 for a total of five times. On days 1, 3, 5, 7, 9, 11, 13 and 15, the body weights of the mice were weighed, and the tumor volumes of the mice were measured. The results are shown in FIG. 3. As can be seen from FIG. 3, compared with the blank group, the treatment with free drug MMAE can inhibit the growth of the tumor to a certain degree, but its inhibitory effect is obviously weaker than that of the polymer-drug conjugate; and the drug-conjugated polymer containing the hydrophilic monomers exhibits a superior tumor inhibitory effect due to its longer blood circulation time.

### Example 2-1: Synthesis of monomer AzMA

The monomer AzMA was obtained by using the same method as that in Example 1-1.

### Example 2-2: Synthesis of FAP-α-responsive monomer MA-N-GP

The monomer MA-N-GP was obtained by using the same method as that in Example 1-3

### Example 2-3: Synthesis of prodrug DBCO-SS-CPT

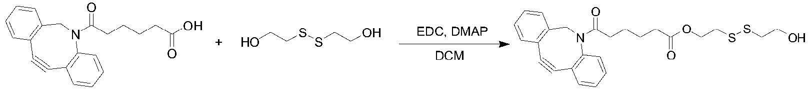

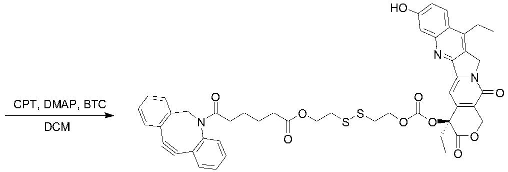

**(1)** (2-hydroxyethyl)disulfide (0.72 mL, 6 mmol) and DBCO-COOH (2 g, 6 mmol) were added to a round-bottom flask, and then 40 mL of anhydrous dichloromethane was added so that the materials were dissolved under stirring in an ice bath. Then, 1-ethyl-3-(3-dimethylaminopropyl)carbodiimide (EDC) (1 g, 6 mmol) was dissolved in 10 mL of anhydrous dichloromethane, and then the solution was added to a constant-pressure dropping funnel. The solution was added dropwise to the round-bottom flask under nitrogen at the rate of 4s/drop, and reacted under stirring at room temperature for 24 h. After completion of the reaction, the reaction product was subjected to suction filtration to remove insoluble substances. The filtrate was concentrated by rotary evaporation, and then was purified by silica gel column chromatography using dichloromethane:methanol = 20:1 (v/v) as a mobile phase. The product fractions were collected and subjected to rotary evaporation to remove the mobile phase, so as to obtain a solid, i.e., DBCO-SS-OH.
**(2)** Under argon, camptothecin (CPT, 1.00 g, 2.87 mmol) and 4-dimethylaminopyridine (DMAP, 1.05 g, 8.62 mmol) were evenly dispersed in 30 mL of anhydrous dichloromethane under sufficient and uniform stirring. In a fume hood, triphosgene (BTC, 0.28 g, 0.96 mmol) was weighed in a round-bottom flask and dissolved in 10 mL of anhydrous dichloromethane, and then the resulting solution was added dropwise to the solution of CPT in dichloromethane. After reacting for 30 min under stirring until the solution turned yellowish-green. The product DBCO-SS-OH (1.39 g, 3.16 mmol) obtained in the previous step was weighed and dissolved in 15 mL of anhydrous dichloromethane, and then the above solution was slowly added dropwise to the round-bottom flask. The mixture reacted under stirring at room temperature for 48 h. After completion of the reaction, the solution turned orange-yellow. The solution was subjected to suction filtration to remove insoluble byproduct salts. The filtrate was concentrated by rotary evaporation. The concentrated filtrate was purified via column chromatography with dichloromethane/methanol = 40:1 (v/v) as a mobile phase. The second fraction, i.e., a product fraction, was collected and subjected to rotary evaporation to remove the mobile phase, so as to obtain a pale yellow solid powder, i.e., DBCO-SS-CPT (1.45 g, yield 73%).

The chemical shifts of DBCO-SS-CPT: ¹H-NMR(CDCl₃), δ(ppm): 0.9-1.1(CH₂CH₃, CCH₃, CCH₃); 1.3-1.5(C(C*H*₃)₃); 1.7-2.4(CCH₂CH₂C, COCCH₂CH₃, CHCH₂, CHCH₂CH₂); 2.8-3.0(SCH₃); 3.2-3.6(NCH₂CH₃, OCH₃); 3.7-4.2(COCH₂NH, COOCH₂, COOCH₂); 4.2-4.6(OCOOCH₂, NHCOCH); 5.2-5.9(NCH₂C,COOCH₂C); 6.0-7.0, 7.5-8.0(NHCO); 7.0-7.7(ph).

### Example 2-4: Synthesis of FAP-α-responsive polymer P(MA-N-GP)-co-P(AzMA)

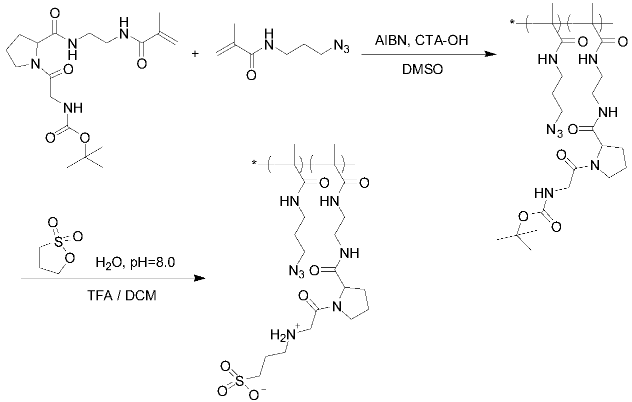

An RAFT polymerization was adopted. MA-N-GP (300 mg, 0.78 mmol), AzMA (40.6 mg, 0.26 mmol) and an initiator azobisisobutyronitrile (AIBN, 2 mg, 0.01 mmol) were weighed and added to the bottom of a Schlenk tube. A dithioester chain transfer agent (CTA-OH, 3.5 mg, 0.01 mmol) was weighed and dissolved in 800 µL of ultra-dry dimethyl sulfoxide (DMSO), and then the dissolved solution was added to the Schlenk tube. All materials were sufficiently dissolved under stirring. Subsequently, the mixture was subjected to three cycles of liquid nitrogen freezing - evacuation for 15 min - argon filling - thawing utilizing a vacuum evacuation device. The reaction tube filled with argon was placed in an oil bath at 70°C for consecutive reaction for 48 h. After completion of the reaction, the reaction solution was added dropwise to 15 mL of ethyl acetate to precipitate a white solid product. The resulting suspension was concentrated, and the supernatant was discarded. The precipitation procedure was repeated twice to remove unreacted monomers and the DMSO solvent. The obtained solid was dried for 24 h to obtain a polymer.

**40** mg of polymer was dissolved in 2 mL of dichloromethane. After complete dissolution, 200 µL of trifluoroacetic acid was added in an ice bath. The mixture reacted under stirring for 1 h to remove a Boc protecting group. After completion of the reaction, the reaction product was subjected to rotary evaporation to remove dichloromethane and trifluoroacetic acid, followed by co-evaporation with dichloromethane twice. The residue was dried in a vacuum oven at room temperature for 4 h, dissolved in 4 mL of ultrapure water under stirring, and then adjusted to pH 8.0 with a saturated sodium bicarbonate solution. Then, 25 mg of propanesultone and 1 mL of ultrapure water were added, and the mixture reacted under stirring in a water bath at 26°C for 24 h. After completion of the reaction, the reaction solution was transferred into a dialysis bag (MWCO = 1000 Da) and dialyzed against ultrapure water for 24 h to remove unreacted propanesultone. The water was changed every 6 h. The solution was lyophilized to obtain a solid powder, i.e., the FAP-α-responsive polymer P(MA-N-GP)-*co*-P(AzMA).

The chemical shift of the P(MA-N-GP)-co-P(AzMA) polymer: ¹H-NMR(CDCl₃), δ(ppm): 0.7-1.0(CH₂CH₃); 1.6-2.1(CCH₂CH₂C, CH₂CH₃); 2.8-3.2(N₃CH₂); 3.2-3.4(N(CH₃)₃); 3.6-3.8(CH₂N(CH₃)₃); 4.0-4.6((SCH₂CH₂)₂, NCOCH, NHCOCH₂); 6.0-7.0(NHCO).

### Example 2-5: Synthesis of CPT-conjugated FAP-α-responsive polymer P(MA-N-GP)-co-P(AzMA-CPT)

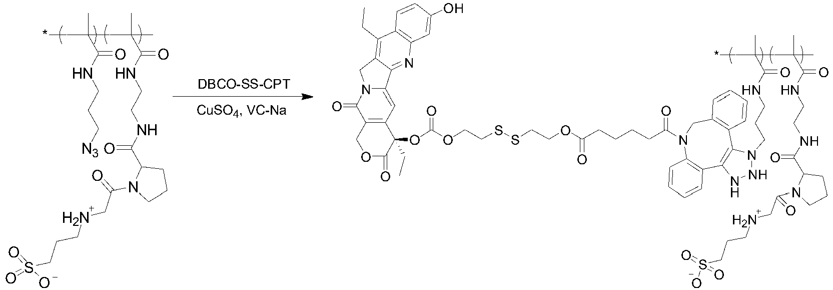

The FAP-α-responsive polymers obtained in Example 2-4 (100 mg, 0.01 mmol), DBCO-SS-CPT (73 mg, 0.1 mmol) and PMDETA (1.772 µg, 20 µL, 0.001 mmol) were dissolved in 0.3 mL of DMF/water mixed solvent (DMF: water = 7:3, v/v). After evacuation twice under argon, copper sulfate pentahydrate and vitamin C (copper sulfate pentahydrate: vitamin C = 1:5, w/w) were added, followed by evacuation three more times. The mixture reacted at 45°C for 48 h. After completion of the reaction, the reaction product was precipitated three times with cold diethyl ether, dialyzed against DMSO and deionized water in sequence, and lyophilized to obtain a product, i.e., the CPT-conjugated FAP-α-responsive polymer P(MA-N-GP)-*co*-P(AzMA-CPT).

The chemical shift of the P(MA-N-GP)-*co*-P(AzMA-CPT) polymer: ¹H-NMR(CDCl₃), δ(ppm): 0.7-1.1(CH₂CH₃, CCH₃, CCH₃); 1.3-1.5(C(C*H*₃)₃); 1.7-2.4(CCH₂CH₂C, COCCH₂CH₃, CHCH₂, CHCH₂CH₂); 2.8-3.2((SCH₂)₂N3CH₂); 3.7-4.2(COCH₂NH, COOCH₂); 4.2-4.6((SCH₂CH₂)₂, OCOOCH₂, NHCOCH); 5.2-5.9(NCH₂, COOCH₂C).

### Example 2-6: Synthesis of polymer P(DS)-co-P(MA-N-GP)-co-P(AzMA)

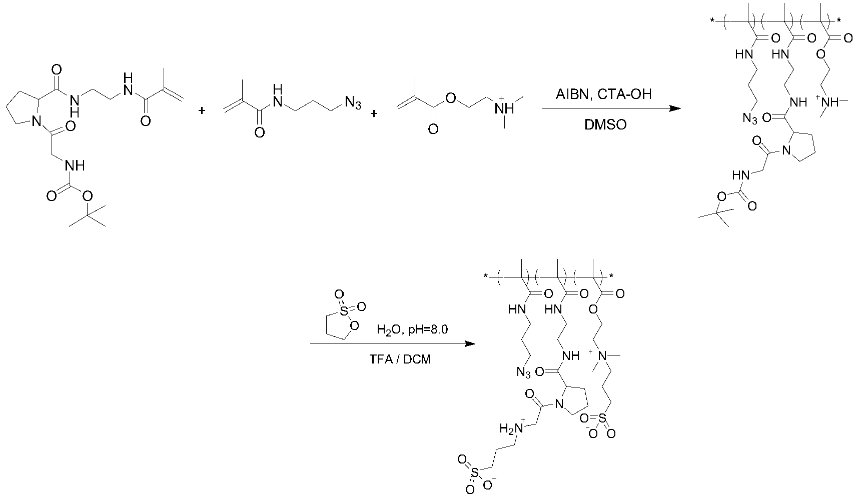

2-(dimethylamino)ethyl methacrylate (DMAEMA) (129 mg, 0.82 mmol), MA-N-GP (209 mg, 0.54 mmol), AzMA (42 mg, 0.27 mmol) and an initiator azobisisobutyronitrile (AIBN, 2 mg, 0.01 mmol) were added to the bottom of a Schlenk tube. A chain transfer agent (CTA-COOH, 3.5 mg, 0.01 mmol) was weighed and dissolved in 1000 µL of ultra-dry dimethyl sulfoxide (DMSO), and the solution was transferred into the Schlenk tube. All materials were sufficiently dissolved under stirring. Subsequently, the mixture was subjected to three cycles of liquid nitrogen freezing - evacuation for 15 min - argon filling - thawing utilizing a vacuum evacuation device. The Schlenk tube filled with argon was placed in an oil bath at 70°C for consecutive reaction for 48 h. After completion of the reaction, the reaction solution was diluted with methanol and then precipitated in cold diethyl ether. After centrifugation, the supernatant was discarded, and the precipitation procedure was repeated twice to remove unreacted monomers and DMSO. The solid was dried for 24 h to obtain a polymer.

40 mg of polymer was dissolved in 2 mL of dichloromethane. After complete dissolution, 200 µL of trifluoroacetic acid was added in an ice bath. The mixture reacted under stirring for 1 h to remove a Boc protecting group. After completion of the reaction, the reaction product was subjected to rotary evaporation to remove dichloromethane and trifluoroacetic acid, followed by co-evaporation with dichloromethane twice. The residue was dried in a vacuum oven at room temperature for 4 h, dissolved in 4 mL of ultrapure water under stirring, and then adjusted to pH 8.0 with a saturated sodium bicarbonate solution. Then, 25 mg of propanesultone and 1 mL of ultrapure water were added, and the mixture reacted under stirring in a water bath at 26°C for 24 h. After completion of the reaction, the reaction solution was transferred into a dialysis bag (MWCO = 1000 Da) and dialyzed against ultrapure water for 24 h to remove unreacted propanesultone. The water was changed every 6 h. The solution was lyophilized to obtain a solid powder, i.e., the FAP-α-responsive polymer P(DS)-*co*-P(MA-N-GP)-*co*-P(AzMA).

The chemical shift of the P(DS)-co-P(MA-N-GP)-co-P(AzMA): ¹H-NMR(CDCl₃), δ(ppm): 0.6-1.2(CH₂CH₃, CCH₃, CCH₃, CCH₃); 1.3-1.5(C(C*H*₃)₃); 1.7-2.4(CHCH₂, CHCH₂CH₂, CH₂CH₃, CCH₂CH₂CCH₂C); 2.8-3.2(NH(CH₃)₂, N₃CH₂); 3.3-3.6(CONHCH₂, (CH₃)₂NHCH₂,CHNCH₂); 3.8-4.1(NCOCH₂); 4.3-4.6((SCH₂CH₂)₂, COOCH₂CH₂NH, NH(CH₃)₂, (COOCH₂CH₂S)₂, CHN); 5.2-5.5(NCH₂C); 5.6-5.8(COOCH₂C).

### Example 2-7: Synthesis of CPT-conjugated FAP-α-responsive polymer P(DS)-co-P(MA-N-GP)-co-P(AzMA-CPT)

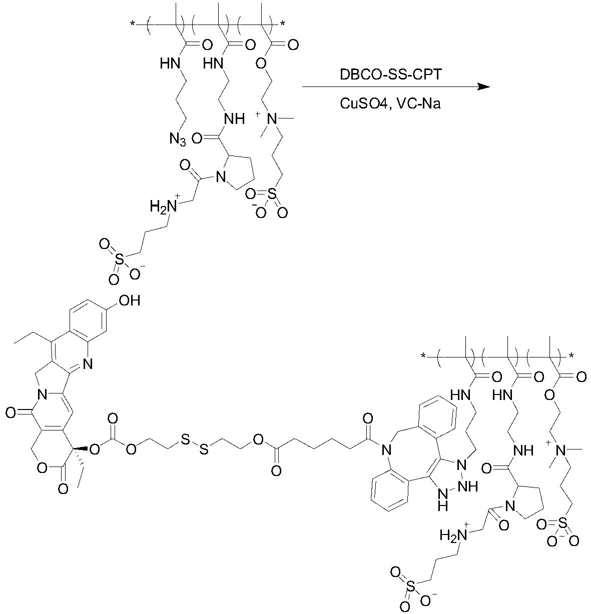

The polymer obtained in Example 2-6 (150 mg, 0.01 mmol), DBCO-SS-CPT (73 mg, 0.1 mmol) and PMDETA (1.772 µg, 20 µL, 0.001 mmol) were dissolved in 0.3 mL of DMF/water mixed solvent (DMF: water = 7:3, v/v). After evacuation twice under argon, copper sulfate pentahydrate and vitamin C (copper sulfate pentahydrate: vitamin C = 1:5, w/w) were added, followed by evacuation three more times. The mixture reacted at 45°C for 48 h. After completion of the reaction, the reaction product was precipitated three times with cold diethyl ether, dialyzed against DMSO and deionized water in sequence, and lyophilized to obtain the CPT-conjugated FAP-α-responsive polymer P(DS)-*co*-P(MA-N-GP)-*co*-P(AzMA-CPT).

The chemical shift of the P(DS)-*co*-P(MA-N-GP)-*co*-P(AzMA-CPT) polymer: ¹H-NMR(CDCl₃), δ(ppm): 0.6-1.2(CH₂CH₃, CCH₃,); 1.3-1.5(C(C*H*₃)₃); 1.7-2.4(CHCH₂, CHCH₂CH₂, CH₂CH₃, CCH₂CH₂CCH₂C); 2.8-3.2(NH(CH₃)₂, S(CH₂)₂, N₃CH₂); 3.3-3.6(CONHCH₂, (CH₃)₂NHCH₂, CHNCH₂); 3.8-4.1(NCOCH₂); 4.3-4.6((SCH₂CH₂)₂, COOCH₂CH₂NH, COOCH₂CH₂NH(CH₃)₂, (COOCH₂CH₂S)₂, CHN); 5.2-5.5(NCH₂C); 5.6-5.8(COOCH₂C).

### Application example 2-1

### (1) Preparation of polymer injection

P(MA-N-GP)-*co*-P(AzMA-CPT) and P(DS)-*co*-P(MA-N-GP)-*co*-P(AzMA-CPT) were dissolved in normal saline separately, and the polymer injections were prepared at a CPT-equivalent concentration of 10 mg/kg body weight, yielding homogeneous, clear and transparent polymer solutions.

### (2) Charge-reversal assay of FAP-α-responsive P(DS)-co-P(MA-N-GP)-co-P(AzMA-CPT)

**A** 50 ng/mL FAP-α solution was prepared using a PBS buffer. 5 mg of polymer (P(DS)-*co*-P(MA-N-GP)-*co*-P(AzMA-CPT)) was dissolved in 5 mL of ultrapure water. The FAP-α solution and the polymer solution were mixed in a volume ratio of 4:1. The mixture reacted in a shaker at 37°C at a rotation speed of 100 rpm. At 0, 0.25, 0.5, 1, 2, 4 and 6 h, 200 µL of reaction solution was collected, filtered through a 0.45 µm membrane filter, and subjected to dynamic light scattering (DLS) to detect Zeta potential changes at different time intervals. As can be seen from FIG. 4, after the polymer is mixed with the FAP-α protein, charge reversal occurs rapidly, and the Zeta potential turns positive.

### (3) Cytotoxicity assay

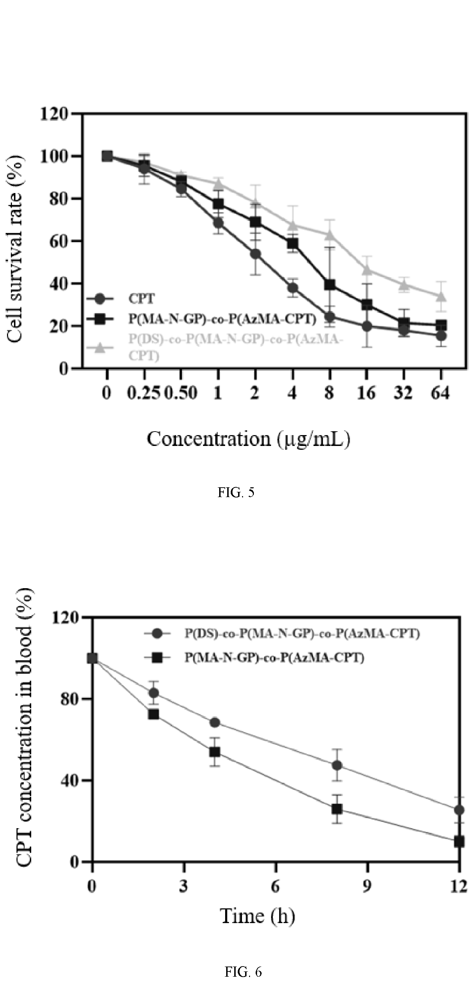

The 4T1 cytotoxicity assay was performed on camptothecin (CPT) and two polymer injections. The results are shown in FIG. 5. As can be seen from FIG. 5, the drug-conjugated polymer containing the hydrophilic monomers, P(DS)-*co*-P(MA-N-GP)-*co*-P(AzMA-CPT), exhibits strong cytotoxicity against 4T1 cells. However, it is difficult to enter the cell membrane due to its resistance to protein adsorption. Therefore, compared with the polymer P(MA-N-GP)-*co*-P(AzMA-CPT) containing no hydrophilic monomers and a free drug (camptothecin), the drug-conjugated polymer containing the hydrophilic monomers has lower cytotoxicity. The cytotoxicity assay results show that both the synthesized two polymer-drug conjugates have a 4T1 killing effect, and possess remarkable cytotoxicity.

### (4) Pharmacokinetic evaluation

2 mL of P(MA-N-GP)-*co*-P(AzMA-CPT) solution obtained in Example 2-5 and 2 mL of P(DS)-*co*-P(MA-N-GP)-*co*-P(AzMA-CPT) solution obtained in Example 2-7 were taken separately, and then 200 µL of 1 mg/mL Cy5.5-alkyne solution was added dropwise therein respectively. The mixture reacted under stirring at room temperature for 24 h. After completion of the reaction, the reaction products were dialyzed against methanol for 3 h and ultrapure water for 24 h in sequence to obtain Cy5.5 fluorescence-labeled polymer solutions, designated as P(MA-N-GP)-*co*-P(AzMA-CPT)^{Cy5.5} and P(DS)-*co*-P(MA-N-GP)-*co*-P(AzMA-CPT)^{Cy5.5}, respectively.

After the mice were acclimated to the environment, 6 mice were divided into two groups with 3 mice per group. The Cy5.5-labeled polymer solutions were intravenously injected into the tail veins of the mice at a dose of 5 mg/kg. Blood samples (approximately 50 µL) were collected from the capillaries around the eyes of the mice after the serum clearance half-life (t₁/₂), and then subjected to in vivo detection before coagulation to determine the fluorescence intensity. The results are shown in FIG. 6. As can be seen from FIG. 6, the t₁/₂ of P(MA-N-GP)-*co*-P(AzMA-CPT) is 4.6 h, and the t₁/₂ of P(DS)-*co*-P(MA-N-GP)-*co*-P(AzMA-CPT) is 7.5 h. The polymerization of the hydrophilic monomers can effectively prevent the micelles from being cleared, thereby achieving the purpose of increasing the blood circulation time and ensuring sufficient long blood circulation.

### (5) Tumor inhibition assay

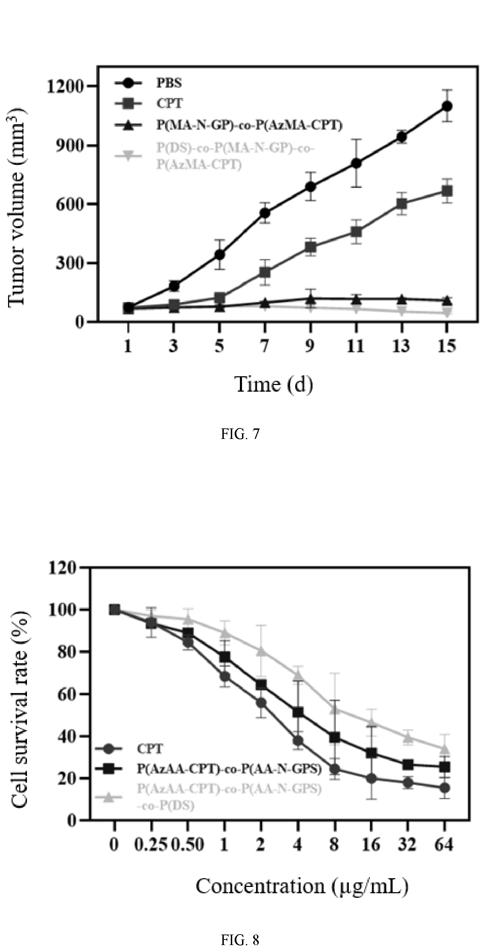

The tumor inhibitory effect of CPT-conjugated polymers in 4T1 breast cancer tumor-bearing mice was investigated. The assay was initiated when the tumor volume reached approximately 100 mm³. The CPT free drug and two drug-conjugated polymer injections were prepared. A PBS buffer was used as a blank group. The drugs were administered via tail vein injection at a CPT-equivalent dose of 5 mg/kg, with an injection volume of 100 µL per mouse each time. The mice were administered on days 1, 4, 7, 10 and 13 for a total of five times. On days 1, 3, 5, 7, 9, 11, 13 and 15, the body weights of the mice were weighed, and the tumor volumes of the mice were measured. The results are shown in FIG. 7. As can be seen from FIG. 7, compared with the blank group, the treatment with free drug CPT inhibits the growth of the tumor to a certain extent, but its inhibitory effect is significantly weaker than that of the polymer-drug conjugates. Due to its longer blood circulation time, the drug-conjugated polymer containing the hydrophilic monomers exhibits a superior tumor inhibition effect.

### Example 3-1: Synthesis of azide monomer

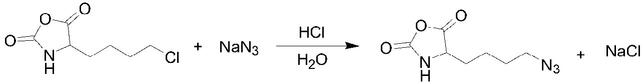

(4S)-4-(4-chlorobutyl)-2,5-oxazolidinedione (1.59 g, 9.2 mmol) was dissolved in 30 mL of deionized water. After complete dissolution, sodium azide (2.25 g, 34.6 mmol) was added to the above solution, and then the mixture reacted at 100°C in the dark for 24 h. After completion of the reaction, the reaction product was slowly cooled to room temperature, and potassium hydroxide was then added until a potassium hydroxide solid was precipitated out, and meanwhile oil droplets were seen on the liquid surface. Subsequently, the mixture was extracted with diethyl ether three times, dried over anhydrous MgSO₄, and concentrated by rotary evaporation to obtain a crude product. The crude product was purified via silica gel column chromatography (a mobile phase: dichloromethane/methanol = 100:1 (V/V)) to obtain the azide monomer.

### Example 3-2: Synthesis of FAP-α-responsive monomer

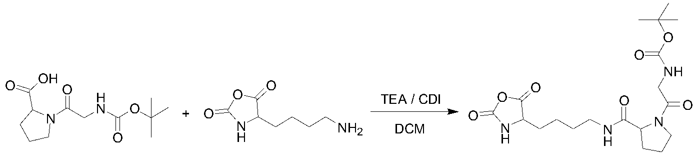

Under argon, Boc-Gly-L-Pro (Boc-GP, 2.50 g, 4.6 mmol) and carbonyldiimidazole (CDI, 1.50 g, 4.6 mmol) were added to a flask, followed by addition of 25 mL of anhydrous dichloromethane, and then the materials were sufficiently dissolved under stirring and then activated at room temperature for 2 h. In a separate flask, (4S)-4-(4-aminobutyl)-2,5-oxazolidinedione (0.95 g, 5.5 mmol) and triethylamine (TEA, 4.7 mL, 16.6 mmol) were added, followed by addition of 30 mL of anhydrous dichloromethane, and then the materials were stirred thoroughly at room temperature for 2 h. The reaction solution activated in the first flask was then added dropwise to the second solution via a constant-pressure dropping funnel, and then the resulting mixture was stirred at room temperature for 24 h. After completion of the reaction, the reaction product was filtered to remove unreacted salts. The remaining product was washed three times with 0.5 M HCl, dried over anhydrous magnesium sulfate, underwent suction filtration and concentrated by rotary evaporation. The concentrated product was purified via silica gel column chromatography using ethyl acetate as a mobile phase. The purified product was subjected to rotary evaporation to remove the mobile phase to obtain a white solid powder (1.6 g, yield 47%).

### Example 3-3: Synthesis of hydrophilic monomer

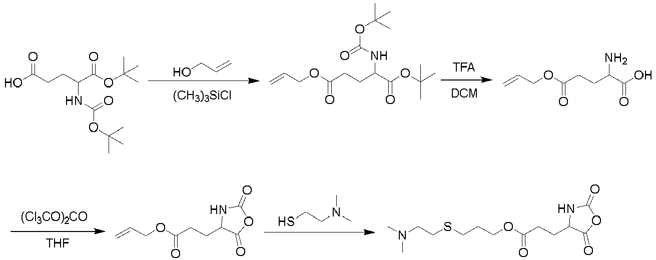

Boc-L-glutamic acid α-tert-butyl ester (5.8 g, 0.029 mol) and allyl alcohol (30 mL, 0.442 mol) were mixed in a 250 mL round-bottom flask. Trimethylsilyl chloride (4 mL, 0.031 mol) was then added to the mixed solution via a syringe. The resulting suspension was heated to 80°C and stirred until the suspension became homogeneous. The solvent was removed in vacuum at 60°C. Diethyl ether (300 mL) was added to generate a precipitate, i.e., a white solid. The solid was filtered and collected, and then recrystallized in ethanol/diethyl ether for further purification to obtain a white solid product.

The above white solid product was dissolved in 10 mL of dichloromethane. After complete dissolution, 3 mL of trifluoroacetic acid was added in an ice bath, and then the mixture reacted under stirring for 12 h to remove the tert-butyl ester protecting group. After completion of the reaction, the reaction product was subjected to rotary evaporation to remove dichloromethane and trifluoroacetic acid, followed by co-evaporation with dichloromethane twice. The residue was dried in a vacuum oven at room temperature for 4 h to obtain an allyl glutamate product.

Under nitrogen, allyl-L-glutamate (2.5 g, 13 mmol), triphosgene (1.9 g, 6.4 mmol) and anhydrous tetrahydrofuran (40 mL) were added to a 250 mL round-bottom flask. The resulting mixture was stirred at room temperature for 24 h, during which allyl glutamate was gradually dissolved. The solvent was removed in vacuum to obtain an oily liquid. The oily liquid was then dissolved in ethyl acetate (20 mL) and washed with a cold saturated aqueous NaHCO₃/H₂O solution. The organic layer was separated, dried over anhydrous MgSO₄ at 0°C and subjected to suction filtration and rotary evaporation to obtain allyl oxazolidinedionepropionate.

The allyl oxazolidinedionepropionate (3 g, 0.014 mol) and aminothiol (1.47 g, 0.014 mol) were dissolved in 20 mL of tetrahydrofuran. The mixture reacted under stirring at 50°C for 2 h. The solvent was removed by rotary evaporation, and the remaining product was dried in vacuum to obtain the NCA-DS monomer.

### Example 3-4: Synthesis of prodrug DBCO-SS-CPT

The prodrug DBCO-SS-CPT was obtained by using the same method as that in Example 2-3.

### Example 3-5: Synthesis of FAP-α-responsive polymer P(AzAA)-co-P(AA-N-GPS)

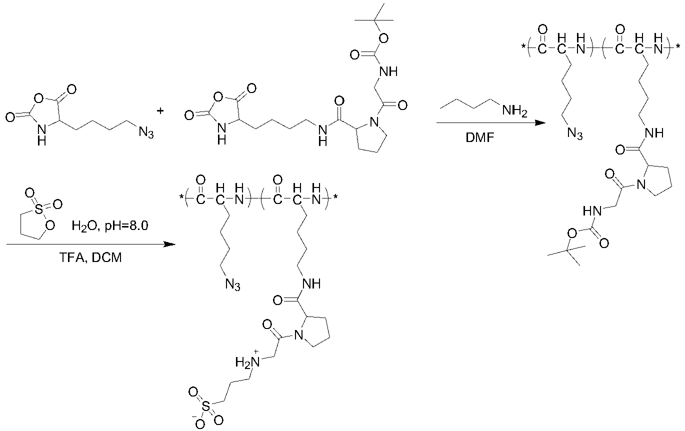

**A** FAP-α-responsive monomer (146 mg, 0.685 mmol) and an azide monomer (73 mg, 0.685 mmol) were dissolved in DMF (5 mL) in a reaction tube. A stock solution of n-butylamine in DMF (CI = 0.253 M, 136 mL, 34.4 mmol) was then added via a syringe. The reaction mixture was stirred at room temperature for 48 h, and then concentrated via rotary evaporation. The concentrated product was dissolved in chloroform, precipitated with cold diethyl ether or methanol, and purified via centrifugation and dialysis to obtain a polymer. The polymer obtained after purification was dried in vacuum to obtain a polymer P(AzAA)-*co*-P(AA-N-GP).

**40** mg of polymer was dissolved in 2 mL of dichloromethane. After complete dissolution, 200 µL of trifluoroacetic acid was added in an ice bath. The mixture reacted under stirring for 1 h to remove a Boc protecting group. After completion of the reaction, the reaction product was subjected to rotary evaporation to remove dichloromethane and trifluoroacetic acid, followed by co-evaporation with dichloromethane twice. The residue was dried in a vacuum oven at room temperature for 4 h, dissolved in 4 mL of ultrapure water under stirring, and then adjusted to pH 8.0 with a saturated sodium bicarbonate solution. Then, 25 mg of propanesultone and 1 mL of ultrapure water were added, and the mixture reacted under stirring in a water bath at 26°C for 24 h. After completion of the reaction, the reaction solution was transferred into a dialysis bag (MWCO = 1000 Da) and dialyzed against ultrapure water for 24 h to remove unreacted propanesultone. The water was changed every 6 h. The remaining product was lyophilized to obtain a solid powder, i.e., the FAP-α-responsive polymer P(AzAA)-*co*-P(AA-N-GPS).

**The** chemical shift of the FAP-α-responsive polymer P(AzAA)-*co*-P(AA-N-GPS): ¹H-NMR(CDCl₃), δ(ppm): 0.7-1.0(CH₂CH₃); 1.6-2.1(CCH₂CH₂C, CH₂CH₃); 2.8-3.2(N(CH₂)₂, N₃CH₂); 3.2-3.4(N(CH₃)₃); 3.6-3.8(CH₂N(CH₃)₃); 4.0-4.4((SCH₂CH₂)₂, NCOCH₂, NHCOCH₂, COOCH₂CH₂O, POCH₂); 6.0-7.0(NHCO).

### Example 3-6: Synthesis of FAP-α-responsive polymer P(AzAA)-co-P(AA-N-GPS)-co-P(DS)

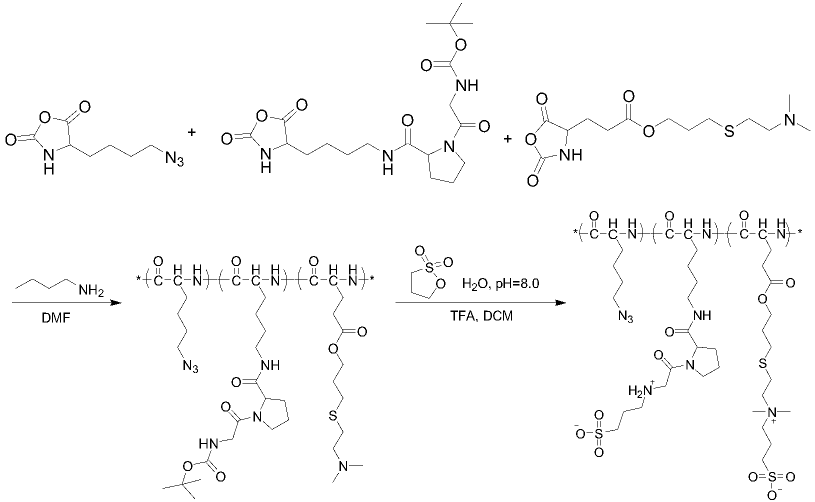

**A** hydrophilic monomer (82 mg, 0.685 mmol), an azide monomer (73 mg, 0.685 mmol) and a FAP-α-responsive monomer (146 mg, 0.685 mmol) were dissolved in DMF (5 mL) in a reaction tube. A stock solution of n-butylamine in DMF (CI = 0.253 M, 136 mL, 34.4 mmol) was then added via a syringe. The reaction mixture was stirred at room temperature for 48 h and concentrated by rotary evaporation. The resulting concentrate was dissolved in chloroform, precipitated with cold diethyl ether or methanol, and the polymer was purified by centrifugation and dialysis. The purified solution was dried in vacuum to obtain a polymer P(AzAA)-*co*-P(AA-N-GP)-*co*-P(DS).

The obtained polymer was dissolved in 3 mL of dichloromethane. After complete dissolution, 300 µL of trifluoroacetic acid was added in an ice bath, and the mixture reacted under stirring for 1 h in the ice bath to remove a Boc protecting group. After completion of the reaction, the reaction product was subjected to rotary evaporation to remove dichloromethane and trifluoroacetic acid, followed by co-evaporation with dichloromethane twice. The residue was dried in a vacuum oven at room temperature for 4 h. The dried product was dissolved in 4 mL of ultrapure water under stirring in a dry flask, and adjusted to pH 8.0 with a saturated sodium bicarbonate solution, so as to obtain the reaction solution. 25 mg of propanesultone and 1 mL of ultrapure water were added, and then the mixture reacted under stirring at 26°C in a water bath for 24 h. After completion of the reaction, the reaction solution was transferred into a dialysis bag (MWCO = 1000 Da) and dialyzed against ultrapure water for 24 h to remove unreacted propanesultone. The water was changed every 6 h. The remaining solution was lyophilized to obtain a solid powder, i.e., the FAP-α-responsive polymer P(AzAA)-*co*-P(AA-N-GPS)-*co*-P(DS).

The chemical shift of the FAP-α-responsive polymer P(AzAA)-*co*-P(AA-N-GPS)-*co*-P(DS): ¹H-NMR(CDCl₃), δ(ppm): 0.6-1.2(CH₂CH₃, CCH₃, CCH₃, CCH₃); 1.3-1.5(C(C*H*₃)₃); 1.7-2.4(CHCH₂, CHCH₂CH₂, CH₂CH₃, CCH₂CH₂CCH₂C); 2.8-3.2(S(CH₂)₂, NH(CH₃)₂, N₃CH₂); 3.3-3.6(CONHCH₂, (CH₃)₂NHCH₂, CHNCH₂); 3.8-4.2(NCOCH₂); 4.2-4.6((SCH₂CH₂)₂, COOCH₂CH₂NH, COOCH₂CH₂NH(CH₃)₂, (COOCH₂CH₂S)₂, CHN); 5.2-5.5(NCH₂C); 5.6-5.8(COOCH₂C).

### Example 3-7: Synthesis of FAP-α-responsive polymer P(AzAA-CPT)-co-P(AA-N-GPS) containing CPT drug linkers

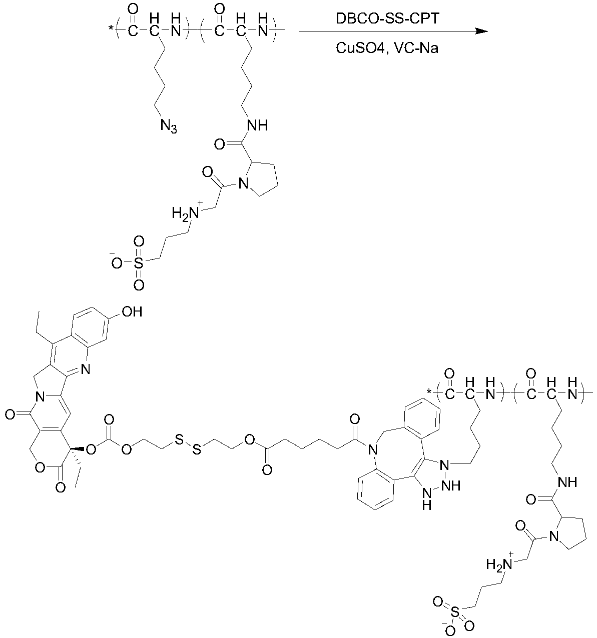

The polymer P(AzAA)-*co*-P(AA-N-GPS) (100 mg, 0.01 mmol), DBCO-SS-CPT (73 mg, 0.1 mmol) and PMDETA (1.772 µg, 20 µL, 0.001 mmol) were dissolved in 0.3 mL of a DMF/water mixed solvent (DMF: water = 7:3 V/V). After evacuation twice under argon, copper sulfate pentahydrate and vitamin C were added (copper sulfate pentahydrate:vitamin C = 1:5 w/w), followed by evacuation three more times. The mixture reacted at 45°C for 48 h. After completion of the reaction, the reaction product was precipitated three times with cold diethyl ether, dialyzed against DMSO and deionized water in sequence, and lyophilized to obtain a product, i.e., the FAP-α-responsive polymer P(AzAA-CPT)-*co*-P(AA-N-GPS) containing the CPT drug linkers.

The chemical shift of the FAP-α-responsive polymer P(AzAA-CPT)-*co*-P(AA-N-GPS) containing the CPT drug linkers: ¹H-NMR(CDCl₃), δ(ppm): 0.7-1.1(CH₂CH₃, CCH₃, CCH₃); 1.3-1.5(C(C*H*₃)₃); 1.7-2.4(CCH₂CH₂C), COCCH₂CH₃, CHCH₂, CHCH₂CH₂); 2.8-3.2((SCH₂)₂, N₃CH₂); 3.7-4.2(COCH₂NH, COOCH₂, COOCH₂); 4.2-4.6((SCH₂CH₂)₂, OCOOCH₂, NCOCH₂, NHCOCH₂); 5.2-5.9(NCH₂C, COOCH₂C).

### Example 3-8: Synthesis of FAP-α-responsive polymer P(AzAA-CPT)-co-P(AA-N-GPS)-co-P(DS) containing CPT drug linkers

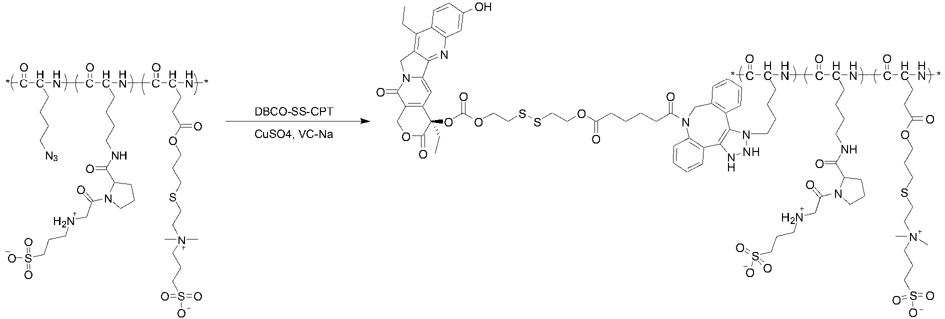

The polymer P(AzAA)-*co*-P(AA-N-GPS)-co-P(DS) (150 mg, 0.01 mmol), DBCO-SS-CPT (73 mg, 0.1 mmol) and PMDETA (1.772 µg, 20 µL, 0.001 mmol) were dissolved in 0.3 mL of a DMF/water mixed solvent (DMF:water = 7:3 V/V). After evacuation twice under argon, copper sulfate pentahydrate and vitamin C were added (copper sulfate pentahydrate: vitamin C = 1:5 w/w), followed by evacuation three more times. The mixture reacted at 45°C for 48 h. After completion of the reaction, the reaction product was precipitated three times with cold diethyl ether, dialyzed against DMSO and deionized water in sequence, and lyophilized to obtain a product, i.e., the FAP-α-responsive polymer P(AzAA-CPT)-co-P(AA-N-GPS)-co-P(DS) containing the CPT drug linker.

The chemical shift of the FAP-α-responsive polymer P(AzAA-CPT)-*co*-P(AA-N-GPS)-*co*-P(DS) containing the CPT drug linker: ¹H-NMR(CDCl₃), δ(ppm): 0.6-1.2(CH₂CH₃, CCH₃, CCH₃, CCH₃); 1.3-1.5(C(C*H*₃)₃); 1.7-2.4(CHCH₂, CHCH₂CH₂, CH₂CH₃, CCH₂CH₂CCH₂C); 2.8-3.2(S(CH₂)₂, NH(CH₃)₂, S(CH₂)₂, N₃CH₂); 3.3-3.6(CONHCH₂, (CH₃)₂NHCH₂, CHNCH₂); 3.8-4.1(NCOCH₂); 4.3-4.6((SCH₂CH₂)₂, COOCH₂CH₂NH, NHCOCH₂, COOCH₂CH₂, N(CH₃)₂, (COOCH₂CH₂S)₂, CHN); 5.2-5.5(NCH₂C); 5.6-5.8(COOCH₂C).

### Application example 3-1

### (1) Preparation of polymer injection

P(AzAA-CPT)-*co*-P(AA-N-GPS) and P(AzAA-CPT)-*co*-P(AA-N-GPS)-*co*-P(DS) were separately dissolved in normal saline, and polymer injections were prepared at a CPT-equivalent concentration of 5 mg/kg body weight to obtain homogeneous, clear and transparent polymer solutions.

### (2) Cytotoxicity assay

4T1 cytotoxicity assay was performed on camptothecin (CPT) and the two polymer injections. The results are shown in FIG. 8. As can be seen from FIG. 8, the drug-conjugated polymer P(AzAA-CPT)-*co*-P(AA-N-GPS)-*co*-P(DS) containing amphiphilic monomers exhibits strong cytotoxicity against 4T1 cells. However, it is difficult to enter the cell membrane due to its resistance to protein adsorption. Therefore, compared with the block polymer P(AzAA-CPT)-*co*-P(AA-N-GPS) (without hydrophilic monomers) and free camptothecin (CPT), it shows lower cytotoxicity. The cytotoxicity assay results show that both the synthesized two polymer-drug conjugates have a 4T1 killing effect, and possess remarkable cytotoxicity.

### (3) Pharmacokinetic evaluation

2 mL of polymer solutions obtained in Example 3-7 and Example 3-8 were taken separately and prepared into solutions. 200 µL of 1 mg/mL Cy5.5-alkyne solution was added dropwise to the above prepared solutions respectively, followed by reacting under stirring at room temperature for 24 h. After completion of the reaction, the mixtures were first dialyzed against methanol for 3 h, followed by dialysis against ultrapure water for 24 h to obtain Cy5.5-labeled polymer solutions.

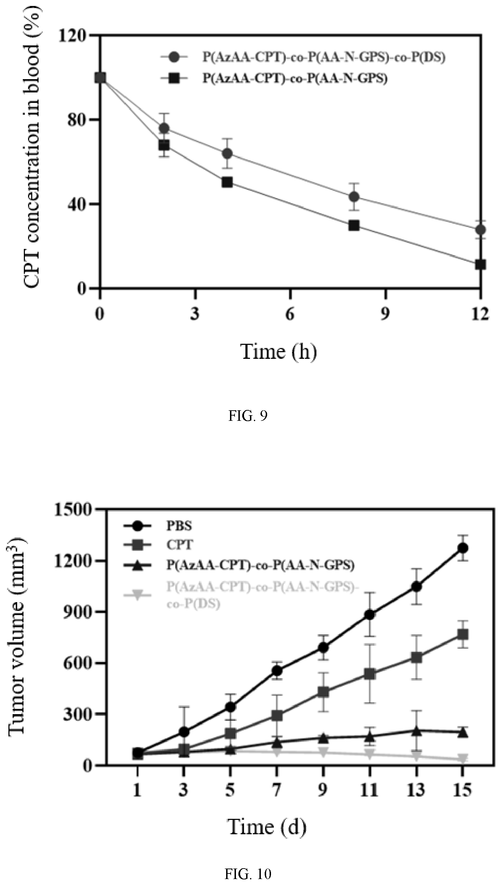

After the mice were acclimated to the environment, 6 mice were divided into two groups with 3 mice per group. The Cy5.5-labeled polymer solutions were intravenously injected into the tail veins of the mice at a dose of 5 mg/kg. After one half-life t₁/₂ of serum clearance, approximately 50 µL of blood was collected from the capillaries around the eyes of mice. The blood samples were immediately subjected to in vivo fluorescence detection before coagulation to determine the fluorescence intensity. The results are shown in FIG. 9. As can be seen from FIG. 9, the t₁/₂ of P(AzAA-CPT)-*co*-P(AA-N-GPS) is 4 h, and the t₁/₂ of P(AzAA-CPT)-*co*-P(AA-N-GPS)-*co*-P(DS) is 6.7 h. The polymerization of the hydrophilic monomers can effectively prevent the micelles from being cleared, thereby achieving the purpose of increasing the blood circulation time and ensuring sufficient long blood circulation.

### (4) Tumor inhibition assay

The tumor inhibitory effect of CPT-conjugated polymers in 4T1 breast cancer tumor-bearing mice was investigated. The assay was initiated when the tumor volume reached approximately 70 mm³. The CPT free drug and two drug-conjugated polymer injections were prepared. A PBS buffer was used as a blank group. The drugs were administered via tail vein injection at a CPT-equivalent dose of 5 mg/kg, with an injection volume of 100 µL per mouse each time. The mice were administered on days 1, 4, 7, 10 and 13 for a total of five times. On days 1, 3, 5, 7, 9, 11, 13 and 15, the body weights of the mice were weighed, and the tumor volumes of the mice were measured. The results are shown in FIG. 10. As can be seen from FIG. 10, compared with the blank group, the free drug CPT can inhibit the growth of the tumor to a certain degree, but its inhibitory effect is obviously weaker than that of the polymer-drug conjugate; and the drug-conjugated polymer containing the hydrophilic monomers exhibits a superior tumor inhibition effect.

Although embodiments of the present disclosure have been shown and described above, it should be understood that the foregoing embodiments are exemplary and shall not be construed as limitations of the present disclosure. Those of ordinary skill in the art may make changes, modifications, substitutions and variations to the foregoing embodiments within the scope of the present disclosure without departing from the principles and spirit of the present disclosure.

## Claims

1. A FAP-α-responsive hydrolysis-triggered charge-reversal polymer-drug conjugate, wherein the FAP-α-responsive hydrolysis-triggered charge-reversal polymer-drug conjugate has the structure represented by Formula (1): 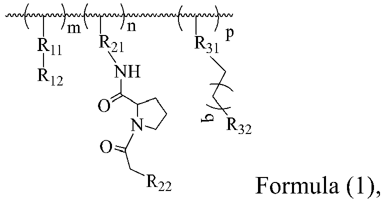 R₁₁ is a linker; R₁₂ is an antitumor drug group; R₂₁ is NH-(CH₂)ₓ-, -O-(CH₂)ₓ-, -CO-(CH₂)ₓ-, -CO-NH-(CH₂)ₓ-, -(CH₂)ₓ-, -(CH₂)ₓ₁O(CH₂)ₓ₂S(CH₂)ₓ₃ or -S-(CH₂)ₓ-, x is 2-6, x1, x2 and x3 are each independently 1-6; R₂₂ is a zwitterionic group; R₃₁ is -NH-, -O-, -OCO-, -NHCO-, -COO-, -CONH-, -S-, -(CH₂)_{z}-O-(CH₂)ₜ-S- or -(CH₂)_{z}-COO-(CH₂)ₜ-S-, q is 1-5, z is 0-5, and t is 1-5; R₃₂ is a zwitterionic group; and m, n and p are molar ratios of three structural units respectively and m:n:p is (5-40%):(10-95%):(0-85%) and m+n+p=100%.

2. The FAP-α-responsive hydrolysis-triggered charge-reversal polymer-drug conjugate according to claim 1, wherein R₁₁ is selected from at least one of the following structures: 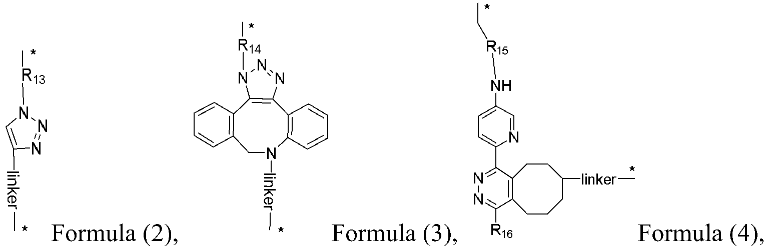 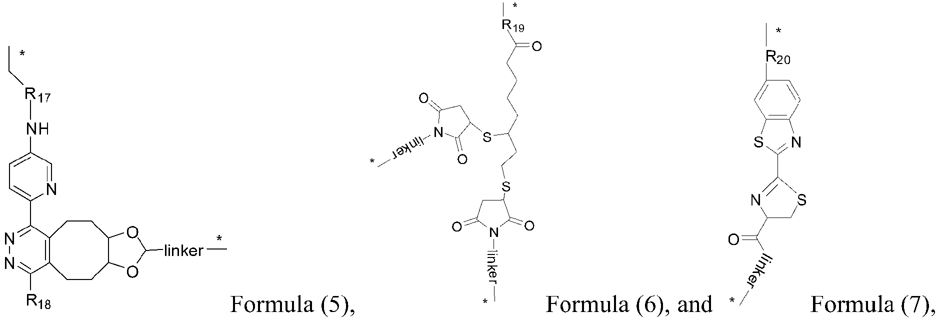
in Formula (2), R₁₃ is an aryl ring, -CO-(CH₂)_{z}-, -O-(CH₂)_{z}-, -NH-(CH₂)_{z}-, -CO-NH-(CH₂)_{z}-, -S-(CH₂)_{z}-, -CO-(CH₂CH₂O)_{z}-, -O-(CH₂CH₂O)_{z}-, -NH-(CH₂CH₂O)_{z}- or -S-(CH₂CH₂O)_{z}-, and z is 1-14;
in Formula (3), R₁₄ is an aryl ring, -CO-(CH₂)_{z}-, -O-(CH₂)_{z}-, -NH-(CH₂)_{z}-, -CO-NH-(CH₂)_{z}-, -S-(CH₂)_{z}-, -CO-(CH₂CH₂O)_{z}-, -O-(CH₂CH₂O)_{z}-, -NH-(CH₂CH₂O)_{z}- or -S-(CH₂CH₂O)_{z}-, and z is 1-14;
in Formula (4), R₁₅ is an aryl ring, -CO-(CH₂)_{z}-CO-, -O-(CH₂)_{z}-CO-, 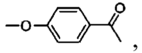 H-(CH₂)_{z}-CO-, -S-(CH₂)_{z}-CO-, -CO-(CH₂CH₂O)_{z}-CO-, -O-(CH₂CH₂O)_{z}-CO-, -NH-(CH₂CH₂O)_{z}-CO- or -S-(CH₂CH₂O)_{z}-CO-, and z is 1-14; and R₁₆ is H or 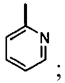
in Formula (5), R₁₇ is an aryl ring, -CO-(CH₂)_{z}-CO-, -O-(CH₂)_{z}-CO-, 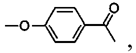 H-(CH₂)_{z}-CO-, -S-(CH₂)_{z}-CO-, -CO-(CH₂CH₂O)_{z}-CO-, -O-(CH₂CH₂O)_{z}-CO-, -NH-(CH₂CH₂O)_{z}-CO- or -S-(CH₂CH₂O)_{z}-CO-, and z is 1-14; and R₁₈ is H or 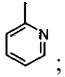
in Formula (6), R₁₉ is an aryl ring, -O-(CH₂)_{z}-O-, -NH-(CH₂)_{z}-NH-, -S-(CH₂)_{z}-NH-, -CO-NH-(CH₂)_{z}- or -S-(CH₂)_{z}-O-, and z is 1-14;
in Formula (7), R₂₀ is an aryl ring, -O-(CH₂)_{z}-O-, -NH-(CH₂)_{z}-O-, -S-(CH₂)_{z}-O-, -CO-NH-(CH₂)_{z}-, -O-(CH₂CH₂O)_{z}-, -NH-(CH₂CH₂O)_{z}- or -S-(CH₂CH₂O)_{z}-, and z is 1-14; and
in the formulas described above, each linker is independently a group having at least one of reduction, reactive oxygen species, pH and enzyme responsiveness.

3. The FAP-α-responsive hydrolysis-triggered charge-reversal polymer-drug conjugate according to claim 1, wherein R₂₂ is selected from at least one of the following structures: 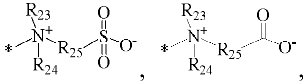 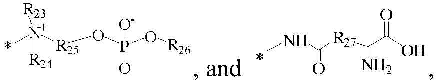 in the formulas described above, R₂₃ and R₂₄ are each independently H or C₁-C₅ alkyl, R₂₅ and R₂₇ are each independently C₁-C₅ alkylene, and R₂₆ is C₁-C₅ alkyl.

4. The FAP-α-responsive hydrolysis-triggered charge-reversal polymer-drug conjugate according to claim 1, wherein R₃₂ is selected from at least one of the following structures: 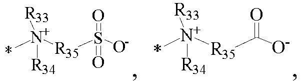 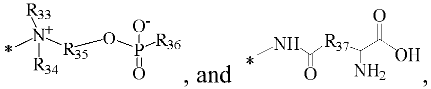 in the formulas described above, R₃₃ and R₃₄ are each independently H or C₁-C₅ alkyl, R₃₅ and R₃₇ are each independently C₁-C₅ alkylene, and R₃₆ is C₁-C₅ alkyl.

5. The FAP-α-responsive hydrolysis-triggered charge-reversal polymer-drug conjugate according to claim 1, wherein the number-average molecular weight of the polymer-drug conjugate is 10,000-80,000 Da; m is 5-40%, n is 10-95%, and p is 0-85%; and the polymer-drug conjugate is a random copolymer or a block copolymer.

6. A preparation method of a FAP-α-responsive hydrolysis-triggered charge-reversal polymer-drug conjugate, comprising modifying a polymer backbone with a prodrug to obtain the polymer-drug conjugate, wherein the polymer backbone has the structure represented by Formula (8): 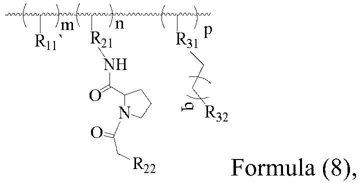 R₁₁' is a linker; R₂₁ is -NH-(CH₂)ₓ-, -O-(CH₂)ₓ-, -CO-(CH₂)ₓ-, -CO-NH-(CH₂)ₓ-, -(CH₂)ₓ-, -(CH₂)ₓ₁O(CH₂)ₓ₂S(CH₂)ₓ₃ or -S-(CH₂)ₓ-, x is 2-6, and x1, x2 and x3 are each independently 1-6; R₂₂ is a zwitterionic group; R₃₁ is -NH-, -O-, -OCO-, -NHCO-, -COO-, -CONH-, -S-, -(CH₂)_{z}-O-(CH₂)ₜ-S- or -(CH₂)_{z}-COO-(CH₂)ₜ-S-, q is 1-5, z is 0-5, and t is 1-5; R₃₂ is a zwitterionic group; and m, n, p are molar ratios of three structural units respectively and m:n:p is (5-40%):(10-95%):(0-85%) and m+n+p=100%.

7. The preparation method of the FAP-α-responsive hydrolysis-triggered charge-reversal polymer-drug conjugate according to claim 6, wherein the modification method is to perform a click chemistry reaction on the R₁₁' in the polymer backbone and the prodrug, and the group for the click chemistry reaction is selected from at least one of azide, trans-cyclooctene, tetrazine, alkyne, dibenzocyclooctyne, bicyclo[6.1.0]nonyne, lipoic acid and maleimide.

8. The preparation method of the FAP-α-responsive hydrolysis-triggered charge-reversal polymer-drug conjugate according to claim 6, wherein R₁₁' is selected from at least one of the following structures: 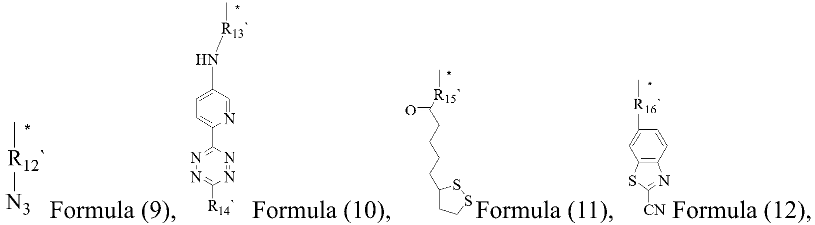
in Formula (9), R₁₂' is an aryl ring, -CO-(CH₂)_{z}-, -O-(CH₂)_{z}-, -NH-(CH₂)_{z}-, -CO-NH-(CH₂)_{z}-, -S-(CH₂)_{z}-, -CO-(CH₂CH₂O)_{z}-, -O-(CH₂CH₂O)_{z}-, -NH-(CH₂CH₂O)_{z}- or -S-(CH₂CH₂O)_{z}-, and z is 1-14;
in Formula (10), R₁₃' is an aryl ring, -CO-(CH₂)_{z}-CO-, -O-(CH₂)_{z}-CO-, 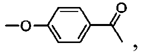 H-(CH₂)_{z}-CO-, -S-(CH₂)_{z}-CO-, -CO-(CH₂CH₂O)_{z}-CO-, -O-(CH₂CH₂O)_{z}-CO-, -NH-(CH₂CH₂O)_{z}-CO- or -S-(CH₂CH₂O)_{z}-CO-, and z is 1-14; and R₁₄' is H or 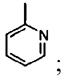
in Formula (11), R₁₅' is an aryl ring, -O-(CH₂)_{z}-O-, -NH-(CH₂)_{z}-NH-, -S-(CH₂)_{z}-NH- or -S-(CH₂)_{z}-O-, and z is 1-14; and
in Formula (12), R₁₆' is an aryl ring, -O-(CH₂)_{z}-O-, -NH-(CH₂)_{z}-O-, -S-(CH₂)_{z}-O-, -CO-NH-(CH₂)_{z}-, -O-(CH₂CH₂O)_{z}-, -NH-(CH₂CH₂O)_{z}- or -S-(CH₂CH₂O)_{z}-, and z is 1-14.

9. The preparation method of the FAP-α-responsive hydrolysis-triggered charge-reversal polymer-drug conjugate according to claim 6, wherein the polymer backbone has at least one of the following structures: 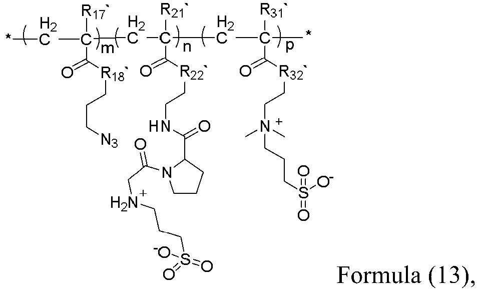 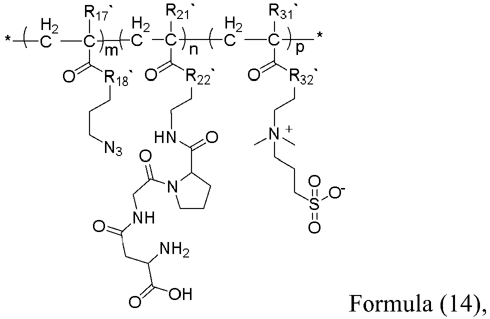 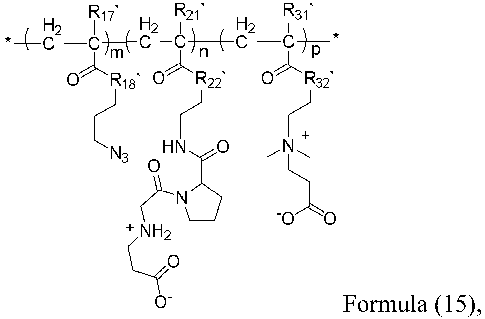 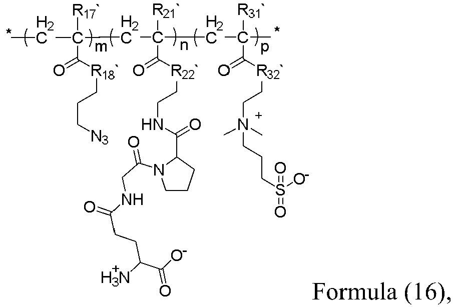 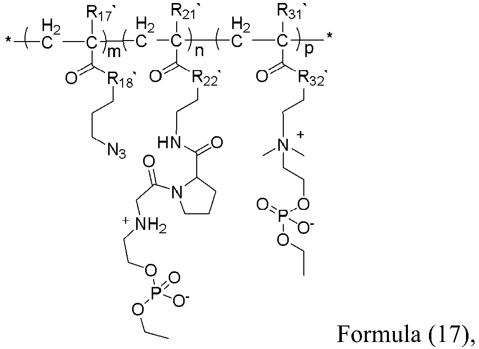 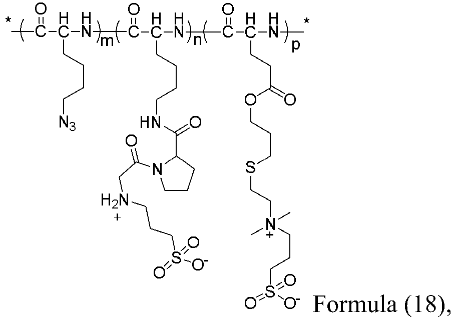 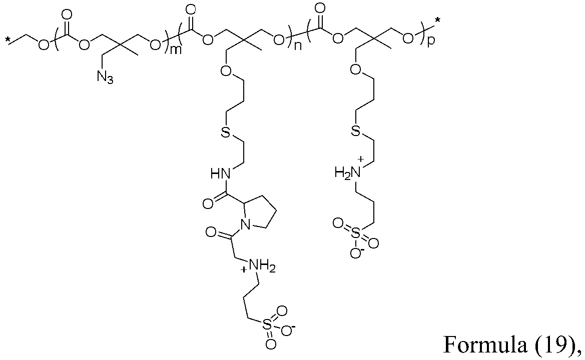 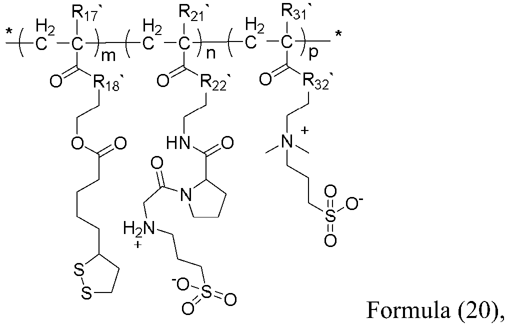 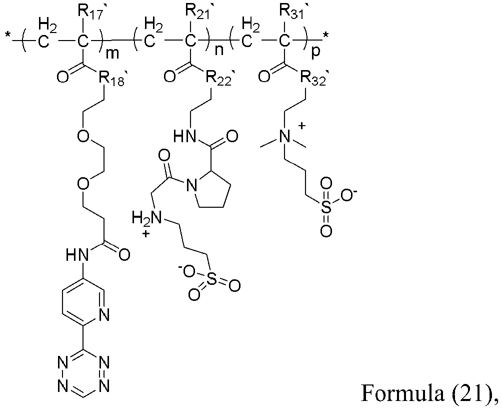 and 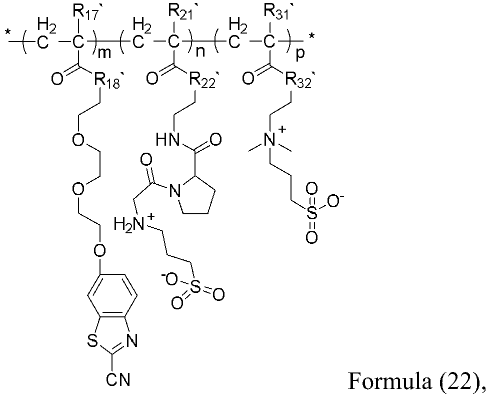 R₁₇', R₂₁' and R₃₁' are each independently H or C₁-C₅ alkyl; and R₁₈', R₂₂' and R₃₂' are each independently O or NH.

10. The preparation method of the FAP-α-responsive hydrolysis-triggered charge-reversal polymer-drug conjugate according to claim 6, wherein the polymer backbone is obtained by random copolymerization or block copolymerization of FAP-α-responsive monomers.

11. The preparation method of the FAP-α-responsive hydrolysis-triggered charge-reversal polymer-drug conjugate according to claim 6, wherein the prodrug has at least one of the following structures: 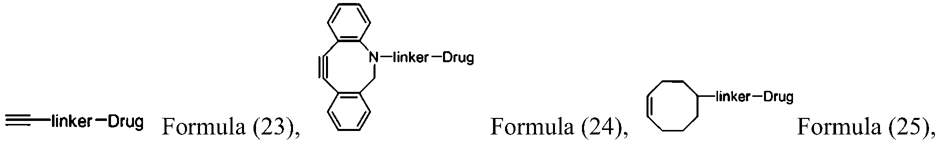 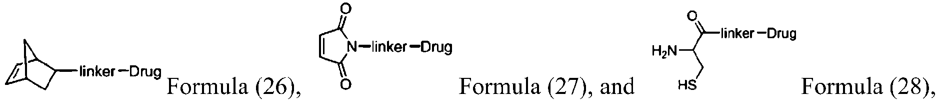 the linker is a group having at least one of reduction, reactive oxygen species, pH and enzyme responsiveness; and Drug is an antitumor drug.

12. The preparation method of the FAP-α-responsive hydrolysis-triggered charge-reversal polymer-drug conjugate according to claim 11, wherein the antitumor drug is selected from at least one of doxorubicin, epirubicin, gemcitabine, cisplatin, carboplatin, paclitaxel, camptothecin, Exatecan, mitomycin C, methotrexate, 7-ethyl-10-hydroxycamptothecin, maytansine, alpha-amanitin, MMAE, MMAF, DM4, calicheamicin, gambogic acid, rhein, vincristine, colchicine, eribulin, Taltobulin, maytansinol, talarostatin A, auristatin E, auristatin F, Piericidin A, ansamitocin P 3, dolastatin 10 and β-Amanitin.

13. The preparation method of the FAP-α-responsive hydrolysis-triggered charge-reversal polymer-drug conjugate according to claim 6, wherein the prodrug has at least one of the following structures: 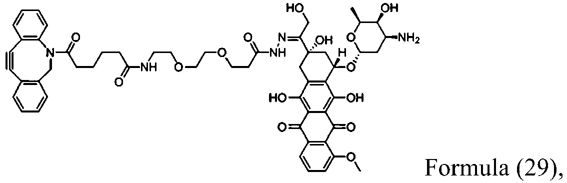 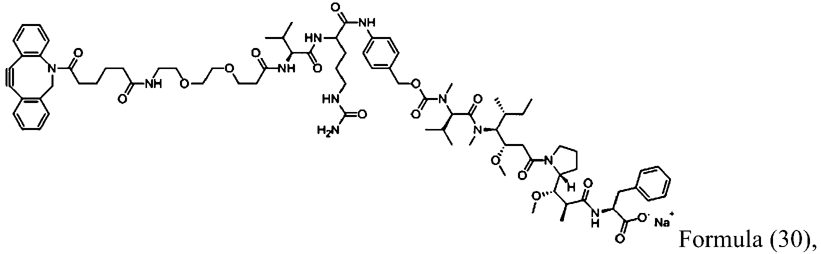 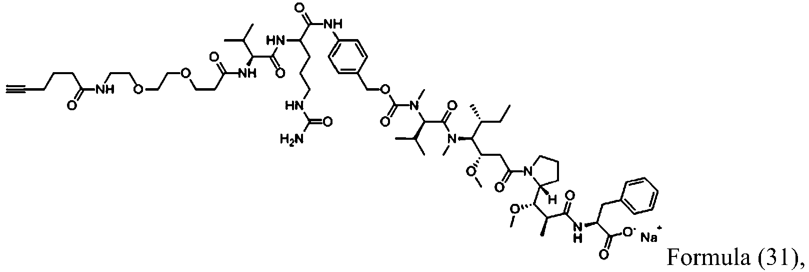 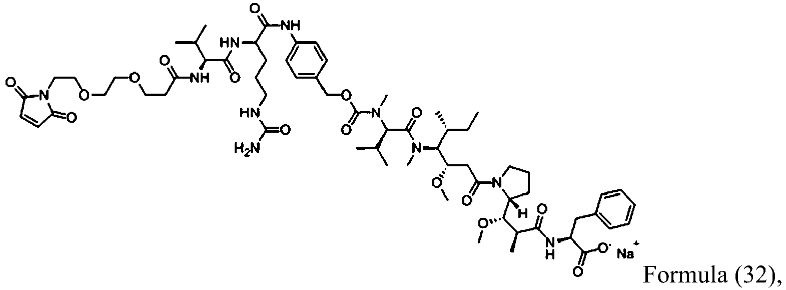 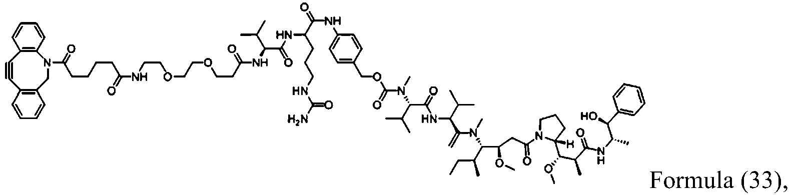 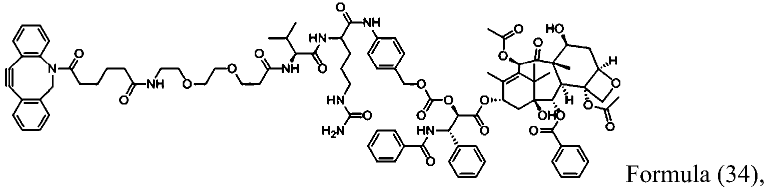 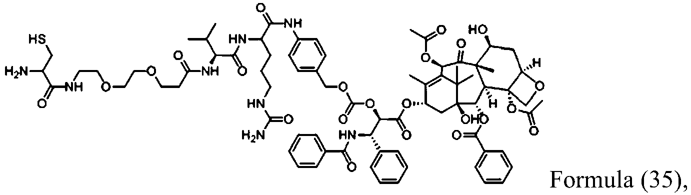 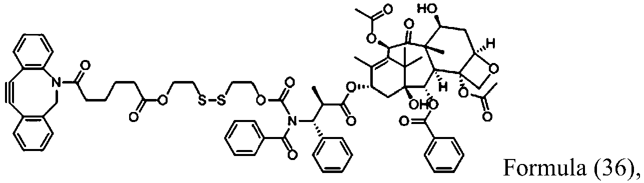 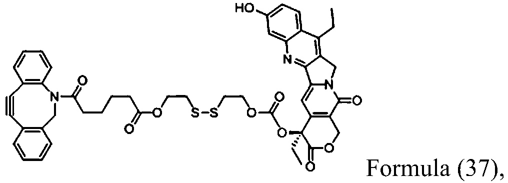 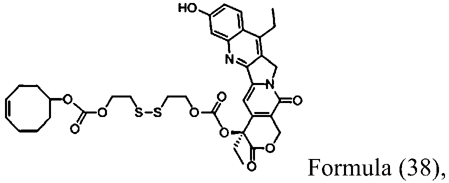 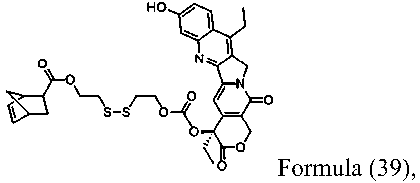 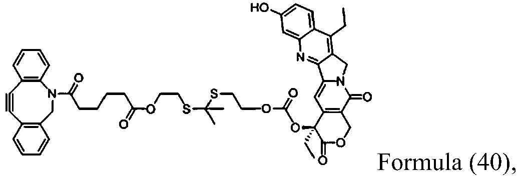 and

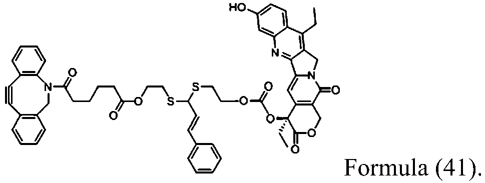

14. The FAP-α-responsive hydrolysis-triggered charge-reversal polymer-drug conjugate prepared by using the method according to claim 6.

15. Use of the FAP-α-responsive hydrolysis-triggered charge-reversal polymer-drug conjugate according to claim 1 in the treatment of tumors.
